# EUROPEAN PATENT APPLICATION

(11) **EP 0 915 155 A1**
(43) Date of publication of application: **12.05.1999**
(21) Application number: 97915700.5
(22) Date of filing: 10.04.1997
(51) Int. Cl.: C12N 15/12, C12P 21/02, C12N 1/21, C12N 5/10, G01N 33/53, C07K 14/435, C07K 16/18, A61K 38/17, A61K 39/395

(54) **NOVEL SIGNAL TRANSDUCER**

(30) Priority: 11.04.1996 JP 113035/96; 25.12.1996 JP 355847/96
(71) Applicant: MOCHIDA PHARMACEUTICAL CO., LTD., Shinjuku-ku Tokyo 160 (JP)
(72) Inventor: INOUE, Jun-ichiro, Tokyo 167 (JP)
(74) Representative: Woods, Geoffrey Corlett
(86) International application number: JP9701236
(87) International publication number: WO9738099

(57) **Abstract**

TRAF5 as a novel protein and a polypeptide as a part thereof; a DNA encoding these; an antisense oligonucleotide against the DNA; an anti-TRAF5 antibody; a vector containing the DNA; a transformant prepared by using the vector; processes for producing the TRAF5 and the polypeptide as a part thereof; methods of screening substances binding to the TRAF5 or the polypeptide, substances regulating the activities of the same, and substances regulating the expression of the same by using the TRAF5 and the polypeptide; novel substances obtained by the screening; and various remedies containing these substances as the active ingredient.

SEQUENCE LISTING

(1) GENERAL INFORMATION:

(i) APPLICANT:
(A) NAME: MOCHIDA PHARMACEUTICAL CO., LTD
(B) STREET: 7, Yotsuya 1-chome, Shinjuku-ku
(C) CITY: Tokyo
(E) COUNTRY: Japan
(F) POSTAL CODE (ZIP): 160

(ii) TITLE OF INVENTION: NOVEL SIGNAL TRANSDUCER

(iii) NUMBER OF SEQUENCES: 12

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: EP 97915700.5

(2) INFORMATION FOR SEQ ID NO: 1:

(i) SEQUENCE CHARACTERISTICS:
(A) LENGTH: 558 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO: 1:

Met Ala His Ser Glu Glu Gln Ala Ala Val Pro Cys Ala Phe Ile Arg
1               5                   10                  15

Gln Asn Ser Gly Asn Ser Ile Ser Leu Asp Phe Glu Pro Asp Thr Glu
            20                  25                  30

Tyr Gln Phe Val Glu Gln Leu Glu Glu Arg Tyr Lys Cys Ala Phe Cys
        35                  40                  45

His Ser Val Leu His Asn Pro His Gln Thr Gly Cys Gly His Arg Phe
    50                  55                  60

Cys Gln Gln Cys Ile Arg Ser Leu Arg Glu Leu Asn Ser Val Pro Ile
65                  70                  75                  80

Cys Pro Val Asp Lys Glu Val Ile Lys Pro Gln Glu Val Phe Lys Asp
                85                  90                  95

Asn Cys Cys Lys Arg Glu Val Leu Asn Leu His Val Tyr Cys Lys Asn
            100                 105                 110

Ala Pro Gly Cys Asn Ala Arg Ile Ile Leu Gly Arg Phe Gln Asp His
        115                 120                 125

Leu Gln His Cys Ser Phe Gln Ala Val Pro Cys Pro Asn Glu Ser Cys
    130                 135                 140

Arg Glu Ala Met Leu Arg Lys Asp Val Lys Glu His Leu Ser Ala Tyr
145                 150                 155                 160

Cys Arg Phe Arg Glu Glu Lys Cys Leu Tyr Cys Lys Arg Asp Ile Val
                165                 170                 175

Val Thr Asn Leu Gln Asp His Glu Glu Asn Ser Cys Pro Ala Tyr Pro
            180                 185                 190

Val Ser Cys Pro Asn Arg Cys Val Gln Thr Ile Pro Arg Ala Arg Val
        195                 200                 205

Asn Glu His Leu Thr Val Cys Pro Glu Ala Glu Gln Asp Cys Pro Phe
    210                 215                 220

Lys His Tyr Gly Cys Thr Val Lys Gly Lys Arg Gly Asn Leu Leu Glu
225                 230                 235                 240

His Glu Arg Ala Ala Leu Gln Asp His Met Leu Leu Val Leu Glu Lys
                245                 250                 255

Asn Tyr Gln Leu Glu Gln Arg Ile Ser Asp Leu Tyr Gln Ser Leu Glu
            260                 265                 270

Gln Lys Glu Ser Lys Ile Gln Gln Leu Ala Glu Thr Val Lys Lys Phe
        275                 280                 285

Glu Lys Glu Leu Lys Gln Phe Thr Gln Met Phe Gly Arg Asn Gly Thr
    290                 295                 300

Phe Leu Ser Asn Val Gln Ala Leu Thr Ser His Thr Asp Lys Ser Ala
305                 310                 315                 320

Trp Leu Glu Ala Gln Val Arg Gln Leu Leu Gln Ile Val Asn Gln Gln
                325                 330                 335

Pro Ser Arg Leu Asp Leu Arg Ser Leu Val Asp Ala Val Asp Ser Val
            340                 345                 350

Lys Gln Arg Ile Thr Gln Leu Glu Ala Ser Asp Gln Arg Leu Val Leu
        355                 360                 365

Leu Glu Gly Glu Thr Ser Lys His Asp Ala His Ile Asn Ile His Lys
    370                 375                 380

Ala Gln Leu Asn Lys Asn Glu Glu Arg Phe Lys Gln Leu Glu Gly Ala
385                 390                 395                 400

Cys Tyr Ser Gly Lys Leu Ile Trp Lys Val Thr Asp Tyr Arg Val Lys
                405                 410                 415

Lys Arg Glu Ala Val Glu Gly His Thr Val Ser Val Phe Ser Gln Pro
            420                 425                 430

Phe Tyr Thr Ser Arg Cys Gly Tyr Arg Leu Cys Ala Arg Ala Tyr Leu
        435                 440                 445

Asn Gly Asp Gly Ser Gly Lys Gly Thr His Leu Ser Leu Tyr Phe Val
    450                 455                 460

Val Met Arg Gly Glu Phe Asp Ser Leu Leu Gln Trp Pro Phe Arg Gln
465                 470                 475                 480

Arg Val Thr Leu Met Leu Leu Asp Gln Ser Gly Lys Lys Asn His Ile
                485                 490                 495

Val Glu Thr Phe Lys Ala Asp Pro Asn Ser Ser Ser Phe Lys Arg Pro
            500                 505                 510

Asp Gly Glu Met Asn Ile Ala Ser Gly Cys Pro Arg Phe Val Ser His
        515                 520                 525

Ser Thr Leu Glu Asn Ser Lys Asn Thr Tyr Ile Lys Asp Asp Thr Leu
    530                 535                 540

Phe Leu Lys Val Ala Val Asp Leu Thr Asp Leu Glu Asp Leu
545                 550                 555

(2) INFORMATION FOR SEQ ID NO: 2:

(i) SEQUENCE CHARACTERISTICS:
(A) LENGTH: 1674 base pairs
(B) TYPE: nucleic acid
(C) STRANDEDNESS: double
(D) TOPOLOGY: linear

(ii) MOLECULE TYPE: cDNA to mRNA

(ix) FEATURE:
(A) NAME/KEY: CDS
(B) LOCATION:1..1674

(xi) SEQUENCE DESCRIPTION: SEQ ID NO: 2:

ATG GCT CAT TCG GAG GAG CAA GCG GCT GTC CCC TGC GCC TTC ATC CGC      48
Met Ala His Ser Glu Glu Gln Ala Ala Val Pro Cys Ala Phe Ile Arg
  1               5                  10                  15

CAG AAC TCT GGC AAC TCA ATT TCC TTG GAC TTT GAG CCC GAC ACC GAG 96
Gln Asn Ser Gly Asn Ser Ile Ser Leu Asp Phe Glu Pro Asp Thr Glu
            20                  25                  30

TAC CAG TTT GTG GAG CAG CTG GAA GAA CGC TAC AAA TGT GCC TTC TGC 144
Tyr Gln Phe Val Glu Gln Leu Glu Glu Arg Tyr Lys Cys Ala Phe Cys
        35                  40                  45

CAC TCC GTG CTT CAC AAC CCC CAC CAG ACC GGC TGC GGG CAC CGC TTC 192
His Ser Val Leu His Asn Pro His Gln Thr Gly Cys Gly His Arg Phe
    50                  55                  60

TGC CAG CAG TGC ATC CGG TCT CTG AGA GAA TTG AAC TCG GTG CCG ATC 240
Cys Gln Gln Cys Ile Arg Ser Leu Arg Glu Leu Asn Ser Val Pro Ile
65                  70                  75                  80

TGC CCG GTA GAC AAG GAG GTC ATC AAG CCT CAG GAG GTG TTC AAA GAC 288
Cys Pro Val Asp Lys Glu Val Ile Lys Pro Gln Glu Val Phe Lys Asp
                85                  90                  95

AAC TGC TGC AAA AGA GAA GTT CTC AAT TTA CAC GTC TAC TGC AAA AAC 336
Asn Cys Cys Lys Arg Glu Val Leu Asn Leu His Val Tyr Cys Lys Asn
            100                 105                 110

GCC CCC GGG TGC AAT GCC AGG ATT ATT CTG GGA CGA TTC CAG GAC CAC 384
Ala Pro Gly Cys Asn Ala Arg Ile Ile Leu Gly Arg Phe Gln Asp His
        115                 120                 125

CTT CAG CAC TGT TCC TTC CAA GCC GTG CCC TGC CCT AAC GAG AGC TGC 432
Leu Gln His Cys Ser Phe Gln Ala Val Pro Cys Pro Asn Glu Ser Cys
    130                 135                 140

CGG GAA GCC ATG CTC CGG AAA GAC GTG AAA GAG CAC CTG AGC GCA TAC 480
Arg Glu Ala Met Leu Arg Lys Asp Val Lys Glu His Leu Ser Ala Tyr
145                 150                 155                 160

TGC CGG TTC CGA GAG GAG AAG TGC CTT TAC TGC AAA AGG GAC ATA GTG 528
Cys Arg Phe Arg Glu Glu Lys Cys Leu Tyr Cys Lys Arg Asp Ile Val
                165                 170                 175

GTG ACC AAC CTG CAG GAT CAT GAG GAA AAC TCG TGT CCT GCG TAC CCA 576
Val Thr Asn Leu Gln Asp His Glu Glu Asn Ser Cys Pro Ala Tyr Pro
            180                 185                 190

GTG TCT TGT CCC AAC AGG TGT GTG CAG ACT ATT CCA AGA GCT AGG GTG 624
Val Ser Cys Pro Asn Arg Cys Val Gln Thr Ile Pro Arg Ala Arg Val
        195                 200                 205

AAT GAA CAC CTT ACT GTA TGT CCT GAG GCT GAG CAA GAC TGT CCC TTT 672
Asn Glu His Leu Thr Val Cys Pro Glu Ala Glu Gln Asp Cys Pro Phe
    210                 215                 220

AAG CAC TAT GGC TGC ACT GTC AAG GGT AAG CGG GGG AAC TTG CTG GAG 720
Lys His Tyr Gly Cys Thr Val Lys Gly Lys Arg Gly Asn Leu Leu Glu
225                 230                 235                 240

CAT GAG CGG GCA GCC CTG CAG GAC CAC ATG CTT CTG GTT TTA GAG AAG 768
His Glu Arg Ala Ala Leu Gln Asp His Met Leu Leu Val Leu Glu Lys
                245                 250                 255

AAC TAC CAA CTA GAA CAG CGG ATC TCT GAT TTA TAT CAG AGT CTC GAA 816
Asn Tyr Gln Leu Glu Gln Arg Ile Ser Asp Leu Tyr Gln Ser Leu Glu
            260                 265                 270

CAG AAG GAA AGC AAG ATC CAG CAG CTG GCA GAA ACC GTG AAG AAG TTC 864
Gln Lys Glu Ser Lys Ile Gln Gln Leu Ala Glu Thr Val Lys Lys Phe
        275                 280                 285

GAA AAG GAG CTT AAG CAG TTC ACA CAG ATG TTT GGC AGA AAT GGA ACT 912
Glu Lys Glu Leu Lys Gln Phe Thr Gln Met Phe Gly Arg Asn Gly Thr
    290                 295                 300

TTC CTC TCA AAT GTC CAG GCT CTC ACC AGT CAC ACG GAC AAG TCA GCT 960
Phe Leu Ser Asn Val Gln Ala Leu Thr Ser His Thr Asp Lys Ser Ala
305                 310                 315                 320

TGG CTG GAA GCG CAG GTG CGG CAG CTG CTA CAA ATA GTT AAC CAG CAG 1008
Trp Leu Glu Ala Gln Val Arg Gln Leu Leu Gln Ile Val Asn Gln Gln
                325                 330                 335

CCA AGT CGA CTT GAT CTG AGG TCT TTG GTG GAT GCG GTT GAC AGC GTG 1056
Pro Ser Arg Leu Asp Leu Arg Ser Leu Val Asp Ala Val Asp Ser Val
            340                 345                 350

AAA CAG AGG ATC ACC CAG CTG GAA GCC AGT GAC CAG AGA TTA GTT CTT 1104
Lys Gln Arg Ile Thr Gln Leu Glu Ala Ser Asp Gln Arg Leu Val Leu
        355                 360                 365

TTA GAG GGG GAG ACC AGC AAG CAC GAC GCA CAC ATT AAT ATC CAC AAA 1152
Leu Glu Gly Glu Thr Ser Lys His Asp Ala His Ile Asn Ile His Lys
    370                 375                 380

GCA CAG CTG AAT AAG AAC GAA GAG CGG TTT AAG CAG CTG GAG GGC GCC 1200
Ala Gln Leu Asn Lys Asn Glu Glu Arg Phe Lys Gln Leu Glu Gly Ala
385                 390                 395                 400

TGC TAC AGT GGC AAG CTC ATC TGG AAG GTG ACA GAT TAC AGG GTG AAG 1248
Cys Tyr Ser Gly Lys Leu Ile Trp Lys Val Thr Asp Tyr Arg Val Lys
                405                 410                 415

AAG AGG GAG GCC GTG GAG GGG CAC ACA GTG TCC GTC TTC AGC CAG CCT 1296
Lys Arg Glu Ala Val Glu Gly His Thr Val Ser Val Phe Ser Gln Pro
            420                 425                 430

TTC TAC ACC AGC CGC TGC GGC TAC CGG CTC TGT GCC AGG GCG TAC CTG 1344
Phe Tyr Thr Ser Arg Cys Gly Tyr Arg Leu Cys Ala Arg Ala Tyr Leu
        435                 440                 445

AAC GGG GAC GGG TCG GGG AAG GGA ACG CAC CTG TCC CTG TAC TTT GTG 1392
Asn Gly Asp Gly Ser Gly Lys Gly Thr His Leu Ser Leu Tyr Phe Val
    450                 455                 460

GTG ATG CGC GGT GAG TTT GAC TCG CTG CTG CAG TGG CCG TTC AGG CAG 1440
Val Met Arg Gly Glu Phe Asp Ser Leu Leu Gln Trp Pro Phe Arg Gln
465                 470                 475                 480

AGG GTG ACC CTG ATG CTT TTG GAC CAG AGC GGC AAG AAG AAC CAT ATT 1488
Arg Val Thr Leu Met Leu Leu Asp Gln Ser Gly Lys Lys Asn His Ile
                485                 490                 495

GTG GAG ACC TTC AAA GCT GAC CCC AAC AGC AGC AGC TTC AAA AGG CCA 1536
Val Glu Thr Phe Lys Ala Asp Pro Asn Ser Ser Ser Phe Lys Arg Pro
            500                 505                 510

GAT GGC GAG ATG AAC ATT GCC TCT GGC TGT CCC CGC TTT GTG TCG CAC 1584
Asp Gly Glu Met Asn Ile Ala Ser Gly Cys Pro Arg Phe Val Ser His
        515                 520                 525

TCT ACT CTG GAG AAC TCC AAG AAC ACC TAC ATT AAA GAC GAC ACA CTG 1632
Ser Thr Leu Glu Asn Ser Lys Asn Thr Tyr Ile Lys Asp Asp Thr Leu
    530                 535                 540

TTC TTG AAA GTG GCC GTG GAT TTA ACT GAC TTG GAG GAT CTG 1674
Phe Leu Lys Val Ala Val Asp Leu Thr Asp Leu Glu Asp Leu
545                 550                 555

(2) INFORMATION FOR SEQ ID NO: 3:

(i) SEQUENCE CHARACTERISTICS:
(A) LENGTH: 2105 base pairs
(B) TYPE: nucleic acid
(C) STRANDEDNESS: double
(D) TOPOLOGY: linear

(ii) MOLECULE TYPE: cDNA to mRNA

(ix) FEATURE:
(A) NAME/KEY: CDS
(B) LOCATION:188..1861

(xi) SEQUENCE DESCRIPTION: SEQ ID NO: 3:

TGTGAGCCGG AGGCGTGTGT GGTAGCGGGC GAACTGAGGC GACGCGGGAC ACCCGCGCCC 60

GGCCGAGGGC ACTTTTGCAA GACTTGTGAG CACAGCCCGT TAACGTGAGC TTAATGCCAG 120

GGTCTCGAGC CTGCGCCGGT GCTATAGCGC GTGCTCGATT GGAAACAGAA CCCGACTCTG 180

CAGAAGA ATG GCT CAT TCG GAG GAG CAA GCG GCT GTC CCC TGC GCC TTC 229
Met Ala His Ser Glu Glu Gln Ala Ala Val Pro Cys Ala Phe
1               5                   10

ATC CGC CAG AAC TCT GGC AAC TCA ATT TCC TTG GAC TTT GAG CCC GAC 277
Ile Arg Gln Asn Ser Gly Asn Ser Ile Ser Leu Asp Phe Glu Pro Asp
15                  20                  25                  30

ACC GAG TAC CAG TTT GTG GAG CAG CTG GAA GAA CGC TAC AAA TGT GCC 325
Thr Glu Tyr Gln Phe Val Glu Gln Leu Glu Glu Arg Tyr Lys Cys Ala
                35                  40                  45

TTC TGC CAC TCC GTG CTT CAC AAC CCC CAC CAG ACC GGC TGC GGG CAC 373
Phe Cys His Ser Val Leu His Asn Pro His Gln Thr Gly Cys Gly His
            50                  55                  60

CGC TTC TGC CAG CAG TGC ATC CGG TCT CTG AGA GAA TTG AAC TCG GTG 421
Arg Phe Cys Gln Gln Cys Ile Arg Ser Leu Arg Glu Leu Asn Ser Val
        65                  70                  75

CCG ATC TGC CCG GTA GAC AAG GAG GTC ATC AAG CCT CAG GAG GTG TTC 469
Pro Ile Cys Pro Val Asp Lys Glu Val Ile Lys Pro Gln Glu Val Phe
    80                  85                  90

AAA GAC AAC TGC TGC AAA AGA GAA GTT CTC AAT TTA CAC GTC TAC TGC 517
Lys Asp Asn Cys Cys Lys Arg Glu Val Leu Asn Leu His Val Tyr Cys
95                  100                 105                 110

AAA AAC GCC CCC GGG TGC AAT GCC AGG ATT ATT CTG GGA CGA TTC CAG 565
Lys Asn Ala Pro Gly Cys Asn Ala Arg Ile Ile Leu Gly Arg Phe Gln
                115                 120                 125

GAC CAC CTT CAG CAC TGT TCC TTC CAA GCC GTG CCC TGC CCT AAC GAG 613
Asp His Leu Gln His Cys Ser Phe Gln Ala Val Pro Cys Pro Asn Glu
            130                 135                 140

AGC TGC CGG GAA GCC ATG CTC CGG AAA GAC GTG AAA GAG CAC CTG AGC 661
Ser Cys Arg Glu Ala Met Leu Arg Lys Asp Val Lys Glu His Leu Ser
        145                 150                 155

GCA TAC TGC CGG TTC CGA GAG GAG AAG TGC CTT TAC TGC AAA AGG GAC 709
Ala Tyr Cys Arg Phe Arg Glu Glu Lys Cys Leu Tyr Cys Lys Arg Asp
    160                 165                 170

ATA GTG GTG ACC AAC CTG CAG GAT CAT GAG GAA AAC TCG TGT CCT GCG 757
Ile Val Val Thr Asn Leu Gln Asp His Glu Glu Asn Ser Cys Pro Ala
175                 180                 185                 190

TAC CCA GTG TCT TGT CCC AAC AGG TGT GTG CAG ACT ATT CCA AGA GCT 805
Tyr Pro Val Ser Cys Pro Asn Arg Cys Val Gln Thr Ile Pro Arg Ala
                195                 200                 205

AGG GTG AAT GAA CAC CTT ACT GTA TGT CCT GAG GCT GAG CAA GAC TGT 853
Arg Val Asn Glu His Leu Thr Val Cys Pro Glu Ala Glu Gln Asp Cys
            210                 215                 220

CCC TTT AAG CAC TAT GGC TGC ACT GTC AAG GGT AAG CGG GGG AAC TTG 901
Pro Phe Lys His Tyr Gly Cys Thr Val Lys Gly Lys Arg Gly Asn Leu
        225                 230                 235

CTG GAG CAT GAG CGG GCA GCC CTG CAG GAC CAC ATG CTT CTG GTT TTA 949
Leu Glu His Glu Arg Ala Ala Leu Gln Asp His Met Leu Leu Val Leu
    240                 245                 250

GAG AAG AAC TAC CAA CTA GAA CAG CGG ATC TCT GAT TTA TAT CAG AGT 997
Glu Lys Asn Tyr Gln Leu Glu Gln Arg Ile Ser Asp Leu Tyr Gln Ser
255                 260                 265                 270

CTC GAA CAG AAG GAA AGC AAG ATC CAG CAG CTG GCA GAA ACC GTG AAG 1045
Leu Glu Gln Lys Glu Ser Lys Ile Gln Gln Leu Ala Glu Thr Val Lys
                275                 280                 285

AAG TTC GAA AAG GAG CTT AAG CAG TTC ACA CAG ATG TTT GGC AGA AAT 1093
Lys Phe Glu Lys Glu Leu Lys Gln Phe Thr Gln Met Phe Gly Arg Asn
            290                 295                 300

GGA ACT TTC CTC TCA AAT GTC CAG GCT CTC ACC AGT CAC ACG GAC AAG 1141
Gly Thr Phe Leu Ser Asn Val Gln Ala Leu Thr Ser His Thr Asp Lys
        305                 310                 315

TCA GCT TGG CTG GAA GCG CAG GTG CGG CAG CTG CTA CAA ATA GTT AAC 1189
Ser Ala Trp Leu Glu Ala Gln Val Arg Gln Leu Leu Gln Ile Val Asn
    320                 325                 330

CAG CAG CCA AGT CGA CTT GAT CTG AGG TCT TTG GTG GAT GCG GTT GAC 1237
Gln Gln Pro Ser Arg Leu Asp Leu Arg Ser Leu Val Asp Ala Val Asp
335                 340                 345                 350

AGC GTG AAA CAG AGG ATC ACC CAG CTG GAA GCC AGT GAC CAG AGA TTA 1285
Ser Val Lys Gln Arg Ile Thr Gln Leu Glu Ala Ser Asp Gln Arg Leu
                355                 360                 365

GTT CTT TTA GAG GGG GAG ACC AGC AAG CAC GAC GCA CAC ATT AAT ATC 1333
Val Leu Leu Glu Gly Glu Thr Ser Lys His Asp Ala His Ile Asn Ile
            370                 375                 380

CAC AAA GCA CAG CTG AAT AAG AAC GAA GAG CGG TTT AAG CAG CTG GAG 1381
His Lys Ala Gln Leu Asn Lys Asn Glu Glu Arg Phe Lys Gln Leu Glu
        385                 390                 395

GGC GCC TGC TAC AGT GGC AAG CTC ATC TGG AAG GTG ACA GAT TAC AGG 1429
Gly Ala Cys Tyr Ser Gly Lys Leu Ile Trp Lys Val Thr Asp Tyr Arg
    400                 405                 410

GTG AAG AAG AGG GAG GCC GTG GAG GGG CAC ACA GTG TCC GTC TTC AGC 1477
Val Lys Lys Arg Glu Ala Val Glu Gly His Thr Val Ser Val Phe Ser
415                 420                 425                 430

CAG CCT TTC TAC ACC AGC CGC TGC GGC TAC CGG CTC TGT GCC AGG GCG 1525
Gln Pro Phe Tyr Thr Ser Arg Cys Gly Tyr Arg Leu Cys Ala Arg Ala
                435                 440                 445

TAC CTG AAC GGG GAC GGG TCG GGG AAG GGA ACG CAC CTG TCC CTG TAC 1573
Tyr Leu Asn Gly Asp Gly Ser Gly Lys Gly Thr His Leu Ser Leu Tyr
            450                 455                 460

TTT GTG GTG ATG CGC GGT GAG TTT GAC TCG CTG CTG CAG TGG CCG TTC 1621
Phe Val Val Met Arg Gly Glu Phe Asp Ser Leu Leu Gln Trp Pro Phe
        465                 470                 475

AGG CAG AGG GTG ACC CTG ATG CTT TTG GAC CAG AGC GGC AAG AAG AAC 1669
Arg Gln Arg Val Thr Leu Met Leu Leu Asp Gln Ser Gly Lys Lys Asn
    480                 485                 490

CAT ATT GTG GAG ACC TTC AAA GCT GAC CCC AAC AGC AGC AGC TTC AAA 1717
His Ile Val Glu Thr Phe Lys Ala Asp Pro Asn Ser Ser Ser Phe Lys
495                 500                 505                 510

AGG CCA GAT GGC GAG ATG AAC ATT GCC TCT GGC TGT CCC CGC TTT GTG 1765
Arg Pro Asp Gly Glu Met Asn Ile Ala Ser Gly Cys Pro Arg Phe Val
                515                 520                 525

TCG CAC TCT ACT CTG GAG AAC TCC AAG AAC ACC TAC ATT AAA GAC GAC 1813
Ser His Ser Thr Leu Glu Asn Ser Lys Asn Thr Tyr Ile Lys Asp Asp
            530                 535                 540

ACA CTG TTC TTG AAA GTG GCC GTG GAT TTA ACT GAC TTG GAG GAT CTG 1861
Thr Leu Phe Leu Lys Val Ala Val Asp Leu Thr Asp Leu Glu Asp Leu
        545                 550                 555

TAGTGTTACC TGATAAGGAA ACTTCTCAGC ACAGGAAAAG GTGTGGCTGT CCCTTGGGCG 1921

CAGCCCTCTG GACTGAGCAG GCTCTTGTTC TTGTCTTCCT GCCTCCGATG TCTGATGTGT 1981

CATCTTTTTA TCTTGGATCC TTCCCTGGTT TGAAACTTTA AACTCTTGAA ATATTGCTGT 2041

TATTTATATT TTTGTATCTT CCAAAAAATT ATAATAATTT GACAACAAAA AAAAAAAAAA 2101

AAAA 2105

(2) INFORMATION FOR SEQ ID NO: 4:

(i) SEQUENCE CHARACTERISTICS:
(A) LENGTH: 557 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO: 4:

Met Ala Tyr Ser Glu Glu His Lys Gly Met Pro Cys Gly Phe Ile Arg
1               5                   10                  15

Gln Asn Ser Gly Asn Ser Ile Ser Leu Asp Phe Glu Pro Ser Ile Glu
            20                  25                  30

Tyr Gln Phe Val Glu Arg Leu Glu Glu Arg Tyr Lys Cys Ala Phe Cys
        35                  40                  45

His Ser Val Leu His Asn Pro His Gln Thr Gly Cys Gly His Arg Phe
    50                  55                  60

Cys Gln His Cys Ile Leu Ser Leu Arg Glu Leu Asn Thr Val Pro Ile
65                  70                  75                  80

Cys Pro Val Asp Lys Glu Val Ile Lys Ser Gln Glu Val Phe Lys Asp
                85                  90                  95

Asn Cys Cys Lys Arg Glu Val Leu Asn Leu Tyr Val Tyr Cys Ser Asn
            100                 105                 110

Ala Pro Gly Cys Asn Ala Lys Val Ile Leu Gly Arg Tyr Gln Asp His
        115                 120                 125

Leu Gln Gln Cys Leu Phe Gln Pro Val Gln Cys Ser Asn Glu Lys Cys
    130                 135                 140

Arg Glu Pro Val Leu Arg Lys Asp Leu Lys Glu His Leu Ser Ala Ser
145                 150                 155                 160

Cys Gln Phe Arg Lys Glu Lys Cys Leu Tyr Cys Lys Lys Asp Val Val
                165                 170                 175

Val Ile Asn Leu Gln Asn His Glu Glu Asn Leu Cys Pro Glu Tyr Pro
            180                 185                 190

Val Phe Cys Pro Asn Asn Cys Ala Lys Ile Ile Leu Lys Thr Glu Val
        195                 200                 205

Asp Glu His Leu Ala Val Cys Pro Glu Ala Glu Gln Asp Cys Pro Phe
    210                 215                 220

Lys His Tyr Gly Cys Ala Val Thr Asp Lys Arg Arg Asn Leu Gln Gln
225                 230                 235                 240

His Glu His Ser Ala Leu Arg Glu His Met Arg Leu Val Leu Glu Lys
                245                 250                 255

Asn Val Gln Leu Glu Glu Gln Ile Ser Asp Leu His Lys Ser Leu Glu
            260                 265                 270

Gln Lys Glu Ser Lys Ile Gln Gln Leu Ala Glu Thr Ile Lys Lys Leu
        275                 280                 285

Glu Lys Glu Phe Lys Gln Phe Ala Gln Leu Phe Gly Lys Asn Gly Ser
    290                 295                 300

Phe Leu Pro Asn Ile Gln Val Phe Ala Ser His Ile Asp Lys Ser Ala
305                 310                 315                 320

Trp Leu Glu Ala Gln Val His Gln Leu Leu Gln Met Val Asn Gln Gln
                325                 330                 335

Gln Asn Lys Phe Asp Leu Arg Pro Leu Met Glu Ala Val Asp Thr Val
            340                 345                 350

Lys Gln Lys Ile Thr Leu Leu Glu Asn Asn Asp Gln Arg Leu Ala Val
        355                 360                 365

Leu Glu Glu Glu Thr Asn Lys His Asp Thr His Ile Asn Ile His Lys
    370                 375                 380

Ala Gln Leu Ser Lys Asn Glu Glu Arg Phe Lys Leu Leu Glu Gly Thr
385                 390                 395                 400

Cys Tyr Asn Gly Lys Leu Ile Trp Lys Val Thr Asp Tyr Lys Met Lys
                405                 410                 415

Lys Arg Glu Ala Val Asp Gly His Thr Val Ser Ile Phe Ser Gln Ser
            420                 425                 430

Phe Tyr Thr Ser Arg Cys Gly Tyr Arg Leu Cys Ala Arg Ala Tyr Leu
        435                 440                 445

Asn Gly Asp Gly Ser Gly Arg Gly Ser His Leu Ser Leu Tyr Phe Val
    450                 455                 460

Val Met Arg Gly Glu Phe Asp Ser Leu Leu Gln Trp Pro Phe Arg Gln
465                 470                 475                 480

Arg Val Thr Leu Met Leu Leu Asp Gln Ser Gly Lys Lys Asn Ile Met
                485                 490                 495

Glu Thr Phe Lys Pro Asp Pro Asn Ser Ser Ser Phe Lys Arg Pro Asp
            500                 505                 510

Gly Glu Met Asn Ile Ala Ser Gly Cys Pro Arg Phe Val Ala His Ser
        515                 520                 525

Val Leu Glu Asn Ala Lys Asn Ala Tyr Ile Lys Asp Asp Thr Leu Phe
    530                 535                 540

Leu Lys Val Ala Val Asp Leu Thr Asp Leu Glu Asp Leu
545                 550                 555

(2) INFORMATION FOR SEQ ID NO: 5:

     (i) SEQUENCE CHARACTERISTICS:
          (A) LENGTH: 1671 base pairs
          (B) TYPE: nucleic acid
          (C) STRANDEDNESS: double
          (D) TOPOLOGY: linear

    (ii) MOLECULE TYPE: cDNA to mRNA

(ix) FEATURE:
(A) NAME/KEY: CDS
(B) LOCATION:1..1671

(xi) SEQUENCE DESCRIPTION: SEQ ID NO: 5:

ATG GCT TAT TCA GAA GAG CAT AAA GGT ATG CCC TGT GGT TTC ATC CGC 48
Met Ala Tyr Ser Glu Glu His Lys Gly Met Pro Cys Gly Phe Ile Arg
1               5                   10                  15

CAG AAT TCC GGC AAC TCC ATT TCC TTG GAC TTT GAG CCC AGT ATA GAG 96
Gln Asn Ser Gly Asn Ser Ile Ser Leu Asp Phe Glu Pro Ser Ile Glu
            20                  25                  30

TAC CAG TTT GTG GAG CGG TTG GAA GAG CGC TAC AAA TGT GCC TTC TGC 144
Tyr Gln Phe Val Glu Arg Leu Glu Glu Arg Tyr Lys Cys Ala Phe Cys
        35                  40                  45

CAC TCG GTG CTT CAC AAC CCC CAC CAG ACA GGA TGT GGG CAC CGC TTC 192
His Ser Val Leu His Asn Pro His Gln Thr Gly Cys Gly His Arg Phe
    50                  55                  60

TGC CAG CAC TGC ATC CTG TCC CTG AGA GAA TTA AAC ACA GTG CCA ATC 240
Cys Gln His Cys Ile Leu Ser Leu Arg Glu Leu Asn Thr Val Pro Ile
65                  70                  75                  80

TGC CCT GTA GAT AAA GAG GTC ATC AAA TCT CAG GAG GTT TTT AAA GAC 288
Cys Pro Val Asp Lys Glu Val Ile Lys Ser Gln Glu Val Phe Lys Asp
                85                  90                  95

AAT TGT TGC AAA AGA GAA GTC CTC AAC TTA TAT GTA TAT TGC AGC AAT 336
Asn Cys Cys Lys Arg Glu Val Leu Asn Leu Tyr Val Tyr Cys Ser Asn
            100                 105                 110

GCT CCT GGA TGT AAT GCC AAG GTT ATT CTG GGC CGG TAC CAG GAT CAC 384
Ala Pro Gly Cys Asn Ala Lys Val Ile Leu Gly Arg Tyr Gln Asp His
        115                 120                 125

CTT CAG CAG TGC TTA TTT CAA CCT GTG CAG TGT TCT AAT GAG AAG TGC 432
Leu Gln Gln Cys Leu Phe Gln Pro Val Gln Cys Ser Asn Glu Lys Cys
    130                 135                 140

CGG GAG CCA GTC CTA CGG AAA GAC CTG AAA GAG CAT TTG AGT GCA TCC 480
Arg Glu Pro Val Leu Arg Lys Asp Leu Lys Glu His Leu Ser Ala Ser
145                 150                 155                 160

TGT CAG TTT CGA AAG GAA AAA TGC CTT TAT TGC AAA AAG GAT GTG GTA 528
Cys Gln Phe Arg Lys Glu Lys Cys Leu Tyr Cys Lys Lys Asp Val Val
                165                 170                 175

GTC ATC AAT CTA CAG AAT CAT GAG GAA AAC TTG TGT CCT GAA TAC CCA 576
Val Ile Asn Leu Gln Asn His Glu Glu Asn Leu Cys Pro Glu Tyr Pro
            180                 185                 190

GTA TTT TGT CCC AAC AAT TGT GCG AAG ATT ATT CTA AAA ACT GAG GTA 624
Val Phe Cys Pro Asn Asn Cys Ala Lys Ile Ile Leu Lys Thr Glu Val
        195                 200                 205

GAT GAA CAC CTG GCT GTA TGT CCT GAA GCT GAG CAA GAC TGT CCT TTT 672
Asp Glu His Leu Ala Val Cys Pro Glu Ala Glu Gln Asp Cys Pro Phe
    210                 215                 220

AAG CAC TAT GGC TGT GCT GTA ACG GAT AAA CGG AGG AAC CTG CAG CAA 720
Lys His Tyr Gly Cys Ala Val Thr Asp Lys Arg Arg Asn Leu Gln Gln
225                 230                 235                 240

CAT GAG CAT TCA GCC TTA CGG GAG CAC ATG CGT TTG GTT TTA GAA AAG 768
His Glu His Ser Ala Leu Arg Glu His Met Arg Leu Val Leu Glu Lys
                245                 250                 255

AAT GTC CAA TTA GAA GAA CAG ATT TCT GAC TTA CAC AAG AGC CTA GAA 816
Asn Val Gln Leu Glu Glu Gln Ile Ser Asp Leu His Lys Ser Leu Glu
            260                 265                 270

CAG AAA GAA AGT AAA ATC CAG CAG CTA GCA GAA ACT ATA AAG AAA CTT 864
Gln Lys Glu Ser Lys Ile Gln Gln Leu Ala Glu Thr Ile Lys Lys Leu
        275                 280                 285

GAA AAG GAG TTC AAG CAG TTT GCA CAG TTG TTT GGC AAA AAT GGA AGC 912
Glu Lys Glu Phe Lys Gln Phe Ala Gln Leu Phe Gly Lys Asn Gly Ser
    290                 295                 300

TTC CTC CCA AAC ATC CAG GTT TTT GCC AGT CAC ATT GAC AAG TCA GCT 960
Phe Leu Pro Asn Ile Gln Val Phe Ala Ser His Ile Asp Lys Ser Ala
305                 310                 315                 320

TGG CTA GAA GCT CAA GTG CAT CAA TTA TTA CAA ATG GTT AAC CAG CAA 1008
Trp Leu Glu Ala Gln Val His Gln Leu Leu Gln Met Val Asn Gln Gln
                325                 330                 335

CAA AAT AAA TTT GAC CTG AGA CCT TTG ATG GAA GCA GTT GAT ACA GTG 1056
Gln Asn Lys Phe Asp Leu Arg Pro Leu Met Glu Ala Val Asp Thr Val
            340                 345                 350

AAA CAG AAA ATT ACC CTG CTA GAA AAC AAT GAT CAA AGA TTA GCC GTT 1104
Lys Gln Lys Ile Thr Leu Leu Glu Asn Asn Asp Gln Arg Leu Ala Val
        355                 360                 365

TTA GAA GAG GAA ACT AAC AAA CAT GAT ACC CAC ATT AAT ATT CAT AAA 1152
Leu Glu Glu Glu Thr Asn Lys His Asp Thr His Ile Asn Ile His Lys
    370                 375                 380

GCA CAG CTG AGT AAA AAT GAA GAG CGA TTT AAA CTG CTG GAG GGT ACT 1200
Ala Gln Leu Ser Lys Asn Glu Glu Arg Phe Lys Leu Leu Glu Gly Thr
385                 390                 395                 400

TGC TAT AAT GGA AAG CTC ATT TGG AAG GTG ACA GAT TAC AAG ATG AAG 1248
Cys Tyr Asn Gly Lys Leu Ile Trp Lys Val Thr Asp Tyr Lys Met Lys
                405                 410                 415

AAG AGA GAG GCG GTG GAT GGG CAC ACA GTG TCC ATC TTC AGC CAG TCC 1296
Lys Arg Glu Ala Val Asp Gly His Thr Val Ser Ile Phe Ser Gln Ser
            420                 425                 430

TTC TAC ACC AGC CGC TGT GGC TAC CGG CTC TGT GCT AGA GCA TAC CTG 1344
Phe Tyr Thr Ser Arg Cys Gly Tyr Arg Leu Cys Ala Arg Ala Tyr Leu
        435                 440                 445

AAT GGG GAT GGG TCA GGG AGG GGG TCA CAC CTG TCC CTA TAC TTT GTG 1392
Asn Gly Asp Gly Ser Gly Arg Gly Ser His Leu Ser Leu Tyr Phe Val
    450                 455                 460

GTC ATG CGA GGA GAG TTT GAC TCA CTG TTG CAG TGG CCA TTC AGG CAG 1440
Val Met Arg Gly Glu Phe Asp Ser Leu Leu Gln Trp Pro Phe Arg Gln
465                 470                 475                 480

AGG GTG ACC CTG ATG CTT CTG GAC CAG AGT GGC AAA AAG AAC ATT ATG 1488
Arg Val Thr Leu Met Leu Leu Asp Gln Ser Gly Lys Lys Asn Ile Met
                485                 490                 495

GAG ACC TTC AAA CCT GAC CCC AAT AGC AGC AGC TTT AAA AGA CCT GAT 1536
Glu Thr Phe Lys Pro Asp Pro Asn Ser Ser Ser Phe Lys Arg Pro Asp
            500                 505                 510

GGG GAG ATG AAC ATT GCA TCT GGC TGT CCC CGC TTT GTG GCT CAT TCT 1584
Gly Glu Met Asn Ile Ala Ser Gly Cys Pro Arg Phe Val Ala His Ser
        515                 520                 525

GTT TTG GAG AAT GCC AAG AAC GCC TAC ATT AAA GAT GAC ACT CTG TTC 1632
Val Leu Glu Asn Ala Lys Asn Ala Tyr Ile Lys Asp Asp Thr Leu Phe
    530                 535                 540

TTG AAA GTG GCC GTG GAC TTA ACT GAC CTG GAG GAT CTC 1671
Leu Lys Val Ala Val Asp Leu Thr Asp Leu Glu Asp Leu
545                 550                 555

(2) INFORMATION FOR SEQ ID NO: 6:

(i) SEQUENCE CHARACTERISTICS:
(A) LENGTH: 3993 base pairs
(B) TYPE: nucleic acid
(C) STRANDEDNESS: double
(D) TOPOLOGY: linear

(ii) MOLECULE TYPE: cDNA to mRNA

(ix) FEATURE:
(A) NAME/KEY: CDS
(B) LOCATION:55..1725

(xi) SEQUENCE DESCRIPTION: SEQ ID NO: 2:

GCAGCAGCCG CGCCTGCAGA CCGGCCTCGC GGAGCCCGCG CGCCGAGCCC CACA ATG 57
Met
1

GCT TAT TCA GAA GAG CAT AAA GGT ATG CCC TGT GGT TTC ATC CGC CAG 105
Ala Tyr Ser Glu Glu His Lys Gly Met Pro Cys Gly Phe Ile Arg Gln
            5                   10                  15

AAT TCC GGC AAC TCC ATT TCC TTG GAC TTT GAG CCC AGT ATA GAG TAC 153
Asn Ser Gly Asn Ser Ile Ser Leu Asp Phe Glu Pro Ser Ile Glu Tyr
        20                  25                  30

CAG TTT GTG GAG CGG TTG GAA GAG CGC TAC AAA TGT GCC TTC TGC CAC 201
Gln Phe Val Glu Arg Leu Glu Glu Arg Tyr Lys Cys Ala Phe Cys His
    35                  40                  45

TCG GTG CTT CAC AAC CCC CAC CAG ACA GGA TGT GGG CAC CGC TTC TGC 249
Ser Val Leu His Asn Pro His Gln Thr Gly Cys Gly His Arg Phe Cys
50                  55                  60                  65

CAG CAC TGC ATC CTG TCC CTG AGA GAA TTA AAC ACA GTG CCA ATC TGC 297
Gln His Cys Ile Leu Ser Leu Arg Glu Leu Asn Thr Val Pro Ile Cys
                70                  75                  80

CCT GTA GAT AAA GAG GTC ATC AAA TCT CAG GAG GTT TTT AAA GAC AAT 345
Pro Val Asp Lys Glu Val Ile Lys Ser Gln Glu Val Phe Lys Asp Asn
            85                  90                  95

TGT TGC AAA AGA GAA GTC CTC AAC TTA TAT GTA TAT TGC AGC AAT GCT 393
Cys Cys Lys Arg Glu Val Leu Asn Leu Tyr Val Tyr Cys Ser Asn Ala
        100                 105                 110

CCT GGA TGT AAT GCC AAG GTT ATT CTG GGC CGG TAC CAG GAT CAC CTT 441
Pro Gly Cys Asn Ala Lys Val Ile Leu Gly Arg Tyr Gln Asp His Leu
    115                 120                 125

CAG CAG TGC TTA TTT CAA CCT GTG CAG TGT TCT AAT GAG AAG TGC CGG 489
Gln Gln Cys Leu Phe Gln Pro Val Gln Cys Ser Asn Glu Lys Cys Arg
130                 135                 140                 145

GAG CCA GTC CTA CGG AAA GAC CTG AAA GAG CAT TTG AGT GCA TCC TGT 537
Glu Pro Val Leu Arg Lys Asp Leu Lys Glu His Leu Ser Ala Ser Cys
                150                 155                 160

CAG TTT CGA AAG GAA AAA TGC CTT TAT TGC AAA AAG GAT GTG GTA GTC 585
Gln Phe Arg Lys Glu Lys Cys Leu Tyr Cys Lys Lys Asp Val Val Val
            165                 170                 175

ATC AAT CTA CAG AAT CAT GAG GAA AAC TTG TGT CCT GAA TAC CCA GTA 633
Ile Asn Leu Gln Asn His Glu Glu Asn Leu Cys Pro Glu Tyr Pro Val
        180                 185                 190

TTT TGT CCC AAC AAT TGT GCG AAG ATT ATT CTA AAA ACT GAG GTA GAT 681
Phe Cys Pro Asn Asn Cys Ala Lys Ile Ile Leu Lys Thr Glu Val Asp
    195                 200                 205

GAA CAC CTG GCT GTA TGT CCT GAA GCT GAG CAA GAC TGT CCT TTT AAG 729
Glu His Leu Ala Val Cys Pro Glu Ala Glu Gln Asp Cys Pro Phe Lys
210                 215                 220                 225

CAC TAT GGC TGT GCT GTA ACG GAT AAA CGG AGG AAC CTG CAG CAA CAT 777
His Tyr Gly Cys Ala Val Thr Asp Lys Arg Arg Asn Leu Gln Gln His
                230                 235                 240

GAG CAT TCA GCC TTA CGG GAG CAC ATG CGT TTG GTT TTA GAA AAG AAT 825
Glu His Ser Ala Leu Arg Glu His Met Arg Leu Val Leu Glu Lys Asn
            245                 250                 255

GTC CAA TTA GAA GAA CAG ATT TCT GAC TTA CAC AAG AGC CTA GAA CAG 873
Val Gln Leu Glu Glu Gln Ile Ser Asp Leu His Lys Ser Leu Glu Gln
        260                 265                 270

AAA GAA AGT AAA ATC CAG CAG CTA GCA GAA ACT ATA AAG AAA CTT GAA 921
Lys Glu Ser Lys Ile Gln Gln Leu Ala Glu Thr Ile Lys Lys Leu Glu
    275                 280                 285

AAG GAG TTC AAG CAG TTT GCA CAG TTG TTT GGC AAA AAT GGA AGC TTC 969
Lys Glu Phe Lys Gln Phe Ala Gln Leu Phe Gly Lys Asn Gly Ser Phe
290                 295                 300                 305

CTC CCA AAC ATC CAG GTT TTT GCC AGT CAC ATT GAC AAG TCA GCT TGG 1017
Leu Pro Asn Ile Gln Val Phe Ala Ser His Ile Asp Lys Ser Ala Trp
                310                 315                 320

CTA GAA GCT CAA GTG CAT CAA TTA TTA CAA ATG GTT AAC CAG CAA CAA 1065
Leu Glu Ala Gln Val His Gln Leu Leu Gln Met Val Asn Gln Gln Gln
            325                 330                 335

AAT AAA TTT GAC CTG AGA CCT TTG ATG GAA GCA GTT GAT ACA GTG AAA 1113
Asn Lys Phe Asp Leu Arg Pro Leu Met Glu Ala Val Asp Thr Val Lys
        340                 345                 350

CAG AAA ATT ACC CTG CTA GAA AAC AAT GAT CAA AGA TTA GCC GTT TTA 1161
Gln Lys Ile Thr Leu Leu Glu Asn Asn Asp Gln Arg Leu Ala Val Leu
    355                 360                 365

GAA GAG GAA ACT AAC AAA CAT GAT ACC CAC ATT AAT ATT CAT AAA GCA 1209
Glu Glu Glu Thr Asn Lys His Asp Thr His Ile Asn Ile His Lys Ala
370                 375                 380                 385

CAG CTG AGT AAA AAT GAA GAG CGA TTT AAA CTG CTG GAG GGT ACT TGC 1257
Gln Leu Ser Lys Asn Glu Glu Arg Phe Lys Leu Leu Glu Gly Thr Cys
                390                 395                 400

TAT AAT GGA AAG CTC ATT TGG AAG GTG ACA GAT TAC AAG ATG AAG AAG 1305
Tyr Asn Gly Lys Leu Ile Trp Lys Val Thr Asp Tyr Lys Met Lys Lys
            405                 410                 415

AGA GAG GCG GTG GAT GGG CAC ACA GTG TCC ATC TTC AGC CAG TCC TTC 1353
Arg Glu Ala Val Asp Gly His Thr Val Ser Ile Phe Ser Gln Ser Phe
        420                 425                 430

TAC ACC AGC CGC TGT GGC TAC CGG CTC TGT GCT AGA GCA TAC CTG AAT 1401
Tyr Thr Ser Arg Cys Gly Tyr Arg Leu Cys Ala Arg Ala Tyr Leu Asn
    435                 440                 445

GGG GAT GGG TCA GGG AGG GGG TCA CAC CTG TCC CTA TAC TTT GTG GTC 1449
Gly Asp Gly Ser Gly Arg Gly Ser His Leu Ser Leu Tyr Phe Val Val
450                 455                 460                 465

ATG CGA GGA GAG TTT GAC TCA CTG TTG CAG TGG CCA TTC AGG CAG AGG 1497
Met Arg Gly Glu Phe Asp Ser Leu Leu Gln Trp Pro Phe Arg Gln Arg
                470                 475                 480

GTG ACC CTG ATG CTT CTG GAC CAG AGT GGC AAA AAG AAC ATT ATG GAG 1545
Val Thr Leu Met Leu Leu Asp Gln Ser Gly Lys Lys Asn Ile Met Glu
            485                 490                 495

ACC TTC AAA CCT GAC CCC AAT AGC AGC AGC TTT AAA AGA CCT GAT GGG 1593
Thr Phe Lys Pro Asp Pro Asn Ser Ser Ser Phe Lys Arg Pro Asp Gly
        500                 505                 510

GAG ATG AAC ATT GCA TCT GGC TGT CCC CGC TTT GTG GCT CAT TCT GTT 1641
Glu Met Asn Ile Ala Ser Gly Cys Pro Arg Phe Val Ala His Ser Val
    515                 520                 525

TTG GAG AAT GCC AAG AAC GCC TAC ATT AAA GAT GAC ACT CTG TTC TTG 1689
Leu Glu Asn Ala Lys Asn Ala Tyr Ile Lys Asp Asp Thr Leu Phe Leu
530                 535                 540                 545

AAA GTG GCC GTG GAC TTA ACT GAC CTG GAG GAT CTC TAGTCACTGT 1735
Lys Val Ala Val Asp Leu Thr Asp Leu Glu Asp Leu
                550                 555

TATGGGGTGA TAAGAGGACT TCTTGGGGCC AGAACTGTGG AGGAGAGCAC ATTTGATTAT 1795

CATATTGACC TGGATTTAGA CTCAAAGCAC ATTTGTATTT GCCTTTTTCC TTAACGTTTG 1855

AAGTCAGTTT AAAACTTCTG AAGTGCTGTC TTTTTACATT TTACTCTGTC CCAGTTTGAA 1915

ACTTAAAACT CTTAGAATAT TCTCTTATTA TTTATATTTT TATATTTCTT GAAAGATGGT 1975

AAGTTTCTTG AAGTTTTTGG GGCGTTTCTC TTTTACTGGT GCTTAGCGCA GTGTCTCGGG 2035

CACTCTAAAT ATTGAGTGTT ATGGAGGACA CAGAGGTAGC AGAATCCCAG TTGAAAATGT 2095

TTTGATATTT TATTGTTTGG CCTATTGATT CTAGACCTGG CCTTAAGTCT GCAAAAGCCA 2155

TCTTTATAAG GTAGGCTGTT CCAGTTAAGA AGTGGGTGAT GTAGTTACAA AGATAATATG 2215

CTCAGTTTGG ACCTTTTTTT CAGTTAAATG CTAAATATAT GAAAATTACT ATACCTCTAA 2275

GTATTTTCAT GAAATTCACC AGCAGTTTGC AAGCACAGTT TTGCAAGGCT GCATAAGAAC 2335

TGGTGAATGG GGTAAGCATT TTCATTCTTC CTGCTGAAGT AAAGCAGAAA GTACTGCATA 2395

GTATATGAGA TATAGCCAGC TAGCTAAAGT TCAGATTTTG TTAGGTTCAA CCCTATGAAA 2455

AAAACTATTT TCATAGGTCA AAAATGGTAA AAAATTAGCA GTTTCATAAG ATTCAACCAA 2515

ATAAATATAT ATATACACAC ACACATACAT ATACACCTAT ATATGTGTGT ATACAAACAG 2575

TTCGAATGTA TTTTGGTGAC AGTAATAAAT CAATGTGAGG ATGGATAGAA TTTAGTATAT 2635

GATAGAGAAA ATGTCATAAA TGGATAAAAG GAATTTACAA CTTGAGGAGA AAACCTTTAC 2695

AATTTCCTAT GGGTGTCAGA AGTACTCTCA GCGAAAACTG ATGGCTAAAA CAGTATCTAC 2755

TATTCTCTGA TAACTTTTTT TTTGAGACAG AGTTTCATTG TCACCCAGGC TGGAGTACAG 2815

TGGCATGATC TCAGCTCACT GCAAACTCTG CCTCCCGAAT TCAAGTGATT CTCCTGCCTC 2875

AGCCTCCTGA GTAGCTGGGA TTACAGGCGC CCGTCACCAC ACCCAGGTAA TTTTTGTATT 2935

TTTAGTAGAG ACGGAGTTTT GCCATGTTGG CCAAGCTGAT CTCAAACTCC TGACCTCAAG 2995

TGATCTGCCC GCCTCGGCCT CCCAAAGTGC TGAGATTACA GGCATGACCC ACCGCGTCAA 3055

GCCTCTGACA ACTATTGAAT TTGTAAGCTG CTATGCAAAT GGGCATTTAT ATAAACTTGT 3115

GATGTTTCTT GTCAGAATTC TGAGTACTCT GTGAAGAACA GAAATGATCA TATTCTTATG 3175

CATCTATCTG TATGGGTCTG AAGGTGTATA TACAAACTGA GATGAGTCCT TATGACTCTT 3235

GATAAGCCTG AGTTTAACAA CAACAAAAAT GCCAAGTTGT CCTGAGCCCT TCTGCGTTGT 3295

TATGCCACTT CCCTACTGCT CATATGCACG CTGGCTCCCC TGGGCACGCA AGGATGAGTA 3355

TGGGCCATGG GCCCCTGTAG AGCTGCTTAC CTGGTGATGA CCATGCACCT TACAATTTCT 3415

GAACAGTTAA CCCTATAGAA GCATGCTTTA TATGAGTGTC TTCTGGGAAG AGGAACCTTC 3475

TTAATCTCTT CTGTGGGATT TTCAAAATGC TAAAGACTCA CACTGCAGCA ATCATCCCAG 3535

ATGATTAAAT TCAAAGAAAT AGGTTCACAA CAGGAATATA CTGAAGAACT AGAGTGTCAC 3595

TGCTGGTGAA CTGTGGCACG GTTGCTCAAC ACATCACCTC GGACAAATTC AGGAAGCATT 3655

TCTTTAGCCC ACAAGTCCAG ACCCAGGTGC TCTGTATGTT TGTTTTTAAT ATTCATCATA 3715

TCCAAGTTCA CTCTGTCTTC CTGAGCAGTG GAAGATCATA TTGCTGTAAC TTCTTTTAAG 3775

TAGTTGATGT GGAAAACATT TTAAAGTGAA TTTGTCAAAA TGCTGGTTTT GTGTTTTATC 3835

CAACTTTTGT GCATATATAT AAAGTATGTC ATGGCATGGT TTGCTTAGGA GTTCAGAGTT 3895

CCTTCATCAT CGAAATAGTG ATTAAGTGAT CCCAGAACAA GGAATACTAG AGTAAAAAGC 3955

ACCTCTTTTT CAGAAAAAAA AAAAAAAAAA AAAAAAAA 3993

(2) INFORMATION FOR SEQ ID NO: 7:

(i) SEQUENCE CHARACTERISTICS:
(A) LENGTH: 34 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "primer"

(xi) SEQUENCE DESCRIPTION: SEQ ID NO: 7:

GCGGATCCTC AAAAAGGTGG TCAAGAAACC AAAG 34

(2) INFORMATION FOR SEQ ID NO: 8:

(i) SEQUENCE CHARACTERISTICS:
(A) LENGTH: 32 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "primer"

(xi) SEQUENCE DESCRIPTION: SEQ ID NO: 8:

GCGTCGACTC AAAAGGTCAG CAAGCAGCCA TC 32

(2) INFORMATION FOR SEQ ID NO: 9:

(i) SEQUENCE CHARACTERISTICS:
(A) LENGTH: 33 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "primer"

(xi) SEQUENCE DESCRIPTION: SEQ ID NO: 9:

CTCCTCGAGA TGGAGTCGAG TAAAAAGATG GAC 33

(2) INFORMATION FOR SEQ ID NO: 10:

(i) SEQUENCE CHARACTERISTICS:

(A) LENGTH: 33 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "primer"

(xi) SEQUENCE DESCRIPTION: SEQ ID NO: 10:

CTTACTAGTT CAGGGATCGG GCAGATCCGA AGT 33

(2) INFORMATION FOR SEQ ID NO: 11:

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "primer"

(xi) SEQUENCE DESCRIPTION: SEQ ID NO: 11:

GCAGCAGCCG CGCCTGCAGA CCGGC 25

(2) INFORMATION FOR SEQ ID NO: 12:

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "primer"

(xi) SEQUENCE DESCRIPTION: SEQ ID NO: 12:

ATCCAGGAGC ATTGCTGCAA TATAC 25

## Description

### Technical Field

This invention relates to a protein which associates with CD40 and transduces CD40-mediated signals, TRAF5 (Tumor Necrosis Factor Receptor-Associated Factor); polypeptides of its domains or any part thereof; DNAs encoding them; antisense oligonucleotides for the DNAs; antibodies against TRAF5 and the polypeptides of its domains; expression vectors comprising said DNAs; transformants by said expression vectors; a process for the preparation of TRAF5 and the polypeptides of its domains using said transformants; a process for the screening of substances which may bind to TRAF5 and the polypeptides of its domains, or may regulate their activity or expression, using TRAF5 and the polypeptides of its domains; and medical compositions for the treatment of various diseases.

### Background Art

After the antigen recognition, B cells will grow clonally and differentiate into antibody-producing cells under the interaction with T cells. It is considered that in the case of no association with antigen-specific T cells, B cells will terminate their growth to be inactivated or induced to apoptosis as a result of self-recognition. It has been discovered that an activity inhibiting the apoptosis exists in CD40-mediated signaling, and it has been suggested that CD40 is deeply involved in the regulation of exclusion mechanism of B cells in peripheral blood (Liu, Y.-J. et al., Nature, 342, 929, 1989, Tubata, T. et al., Nature, 364, 645, 1993). Furthermore, it has been revealed that CD40-mediated signaling may play an essential role in isotype switching of immunoglobulins, the germinal center formation and affinity maturation of antibodies (Banchereau, J., et al., Annu. Rev. Immunol., 12, 881, 1994). It is also known that the CD40-mediated signaling can induce the expression of CD23, a low-affinity IgE receptor (Cheng, G., et al., Science, 267, 1994), and that the CD40-mediated signaling is involved in the activation of a transcription factor, NFkB (Berberich, I., et al., J. Immunol., 153, 4357, 1994).

CD40 is expressed not only in B cells, but also in their precursors, activated macrophage/monocyte, follicular dendric cells, Langerhans cells, thymus-epithelial cells and various cancer cells (Banchereau, J., et al., Annu. Rev. Immunol., 12, 881, 1994). It is suggested that the CD40-mediated signaling is not only essential for the activation, growth and differentiation of B cells, but also is involved in antitumor activity, the cytokines production, and the T cells activation.

CD40 has four cysteine-rich motifs in an extracellular domain and is an type-I membrane protein which belongs to NGFR family, like TNFR-1, 2 (Tumor Necrosis Factor Receptor-1, 2), Fas, OX40 and CD30.

It was reported that CD40 ligand (CD40L) was present on the activated T cells (Armitage R. J. et al., Nature, 357, 80, 1992), and has been considered that CD40-CD40L system is a crucial information-transducing mechanism in the association of B cells and T cells.

Recently, TRAF1 and TRAF2 with a TRAF (Tumor Necrosis Factor Receptor-Associated Factor) domain have been identified as a signal transducer which associates with the intracellular domain of TNFR-2. On the other hand, CD40bp, LAP-1 and CRAF1, also known as TRAF3, have been identified as a signal transducer which associates with the intracellular domain of CD40 (CD40 Receptor-Associated Factor; Cheng et al., Science, 267, 1494, 1995).

The present inventor has now succeeded in cloning of the gene for a novel signal transducer, mouse TRAF5 (which is the same substance as that identified as "CRAF2" in the specification of the priority application of the present application, which was filed on April 11, 1996 (the Japanese Patent Application Hei 8 (1996)-113035), by means of a two-hybrid screening using the intracellular domain protein of mouse CD40. The novel signal transducer associates with the intracellular domain of CD40, but not with that of TNFR-2. Further, cloning of the gene for human TRAF5 has been completed based on the sequence of mouse TRAF5 to lead the present invention.

### Disclosure of Invention

The present invention relates to the novel protein TRAF5, a signal transducer which associates with the intracellular domain of CD40.

The present invention relates also to the novel protein TRAF5, a signal transducer which associates with the intracellular domain of CD40, but not with that of TNFR-2.

The present TRAF5 has no limitation with respect to its origin. The examples of the present TRAF5 are that of mouse and human, which may be characterized by an amino acid sequence of the SEQ ID No.1 or No.4 in the Sequence Listing, or their partial sequences.

It should be noted that the above amino acid sequences are only the examples of the present TRAF5, and that the present TRAF5 includes any polypeptides which have an amino acid sequence different partially from the above sequences due to deletion, substitution, addition, etc. as long as they may associate with the intracellular domain of CD40, and which may or may not associate with that of TNFR-2. TRAF5 conjugated with sugar chains, polyethylene glycol, etc. and that fused with other proteins may also be included in the present TRAF5 as long as they possess the activity of TRAF5. The present TRAF5 is different from TRAF1, TRAF2 and CRAF1 with the TRAF domain which associates with the intracellular domain of TNFR-2 or CD40. It is considered that any substance with an amino acid sequence having a high homology to the above amino acid sequences, which has the characteristics of associating with the intracellular domain of CD40, or which has the characteristics of associating with the intracellular domain of CD40 but not with the intracellular domain of TNFR-2, may possess the function of TRAF5. Accordingly, the TRAF5 of the present invention may include the substance with an amino acid sequence having such a high homology as about 60 % or more, especially 80 % or more to the above amino acid sequences or any part thereof, that shows properties similar to mouse or human TRAF5 Human RAF5 is preferred for use in a medical composition, as mentioned later.

The present TRAF5 is an intracellular protein, consisting of a RING finger domain, Zn finger domain, coiled-coil domain and TRAF-C domain.

The present invention therefore relates also to a polypeptide comprising at least each of the above domains or any part thereof, or to any combination of said polypeptides.

The RING finger domain, Zn finger domain, coiled-coil domain and TRAF-C domain correspond to the amino acids No. 45-84, No. 110-249, No.251-403 and No. 404-558, respectively, of the SEQ ID No.1 in the Sequence Listing, or to the amino acids No.45-84, No. 110-249, No.251-403 and No.404-557, respectively, of the SEQ ID No.4 of the Sequence Listing. These amino acid sequences, however, are the only examples of the present polypeptides. The present polypeptide includes any polypeptides which have an amino acid sequence different partially from the above ones due to deletion, substitution, addition , etc. as long as they may have the same function as any one of the above domains. Similarly, the boundaries between the domains should not be fixed to those of the above domains, and polypeptides which contain a region exceeding said boundaries in the direction of an amino- or carboxyl-terminus or both of them by further a few or ten-odd amino acids may be also included in the polypeptides of the present invention.

B cells producing an antibody against a self-antigen are usually eliminated by apoptosis, but signaling from helper T cells will rescue B cells from such apoptosis and induce them to differentiate into antibody-producing cells. The present TRAF5 and polypeptide of its part may be therefore used as a medicament to treat autoimmune disease by regulating the transduction of CD40-mediated signals.

B cells produce IgM antibody at first, but will produce IgG, IgA and IgE antibodies upon Ig isotype switching induced by CD40-mediated signaling. IgE antibody is very easily produced in allergy patients. As one of the reasons is possibly enhancement of the Ig isotype switching, the present TRAF5 and polypeptide of its part may be used as a medicament to treat allergy by regulating the transduction of CD40-mediated signals so as to inhibit the exasperation of the production of IgE.

Furthermore, since CD40-mediated signaling is involved in antitumor activity, various immuno reactions such as the production of cytokines and activation of T cells, and immune diseases. The present TRAF5 and polypeptide of its part may be therefore used as a medicament with cell growth-inhibiting activity, or a medicament for the treatment of various immune diseases by regulating the transduction of CD40-mediated signals.

The present TRAF5 and polypeptide of its part may be introduced into a target cell, for example, being encapsulated in a liposome.

The present invention also relates to a DNA comprising the base sequence encoding the amino acid sequence of the present TRAF5 or its polypeptide part. The present DNAs include any type of DNA such as a genomic DNA and cDNA. The present cDNA may be prepared from mouse testis cDNA library, T cell lymphoma cDNA library, human B cell lymphoma and the like by the known methods such as colony hybridisation, plaque hybridization and PCR. The two-hybrid screening method may be used as well (Mosialos G., et al., Cell 80, 389, 1995). It is also possible to use cDNA libraries prepared from lung, thymus, spleen or kidney.

The examples of the present base sequences are illustrated as the SEQ ID No.2 and SEQ ID No.5 in the Sequence Listing. As described in the following examples, the DNAs of the SEQ ID No.3 and SEQ ID No.6 in the Sequence Listing are inserted into a plasmid vector, and *Escherichia coli* strains transformed with the vector have been deposited in the National Institute of Bioscience and Human-Technology, Agency of Industrial Science and Technology.

The present DNA include DNAs which comprise any other base sequences encoding the same amino acid sequence as the above, and which may be prepared by a chemical synthesis method or genetic engineering method in consideration of degeneracy of a genetic code.

Furthermore, as mentioned in the above, it is considered that the DNA encoding the polypeptide with an amino acid sequence having a high homology to TRAF5 or its polypepetide part may hybridize with the DNA of the present invention.

Accordingly, the present DNA includes DNAs which may hybridize with the base sequences shown as the SEQ ID No.2 and SEQ ID No.5 in the Sequence Listing under a stringent condition, and their DNA fragments.

The present DNA may be used for the production of TRAF5 or its polypeptide part by the genetic engineering method. It may be inserted into a suitable vector and also utilized in gene therapy. Further, transgenic animals and knock-out animals may be prepared based on these base sequences.

Also the present invention relates to an antisense oligonucleotide and its derivatives for the present DNAs. The present antisense oligonucleotides and their derivatives may be complementarily bound to mRNA encoding the present TRAF5 or the polypeptide comprising each domain of TRAF5 or to their part so as to block their expression by inhibiting the translation of these mRNA into polypeptides.

The present antisense oligonucleotides and their derivatives include those binding to the base sequences encoding TRAF5, and those binding to non-coding regions upstream or downstream of TRAF5 as well.

The present antisense oligonucleotides and their derivatives have the base sequences complementary to the present DNA or its part. Thus, they may have a chain complementary to, for example, the DNA shown as the SEQ ID No. 2, No.3, No.5 or No.6 in the Sequence Listing or their parts. Such complementary chain may contain Uracil (U) instead of Thymine (T) as a base complementary to Adenine (A).

The present antisense oligonucleotides derivatives further include any substances which are similar to an oligonucleotide in steric structure and function, such as those in which other substances are bound to 3'- or 5'-terminus of the oligonucleotide; those in which at least one of base, sugar and phosphoric acid is replaced or modified; those containing non-naturally-occurring base, sugar or phosphoric acid; and those having a backbone other than that of sugar-phosphoric acid.

The present antisense oligonucleotides and their derivatives may be prepared by the known methods (for example, Stanley T. Crooke and Bernald Lebleu ed., in Antisense Research and Applications, CRC Publishing, Florida, 1993). The derivatives such as those of methyl phosphonate type or of phosphorothionate type may be prepared using a chemical synthesizer (394 type of Perkin Elmer Japan Co. Ltd., for example). In such case, the operations should be made in accordance with the instruction attached thereto and the synthesized products may be purified by a reverse HPLC chromatography method, for example, to obtain the present antisense oligonucleotides and their derivatives.

The present antisense oligonucleotides and their derivatives may be labelled with a radioisotope, fluorescent substance, enzyme or luminescent substance to use in the detection or determination of DNA or RNA encoding the present TRAF5 or its polypeptide part in a sample.

When the present antisense oligonucleotides and their derivatives are applied to medicaments, it is preferable to use those with a pharmaceutically suitable purity and in a pharmaceutically acceptable way.

The present antisense oligonucleotides and their derivatives may be used as a medicament for the treatment of allergy by regulating the transduction of CD40-mediated signals to inhibit the enhancement of the production of IgE.

The present antisense oligonucleotides and their derivatives may be used also as a medicament with cell growth-inhibiting activity, or as a medicament for the treatment of various immune diseases such as autoimmune disease by regulating the transduction of CD40-mediated signals.

The present antisense oligonucleotides and their derivatives may be used in the form of solution or suspension in a suitable solvent, or encapsulated in a liposome or inserted into a suitable vector.

Furthermore, this invention relates to an antibody recognizing the present TRAF5 or its part.

The present antibodies include ones which may cross-react with TRAF-1, TRAF-2, CRAF1 or their polypeptide parts in addition to ones which specifically recognize TRAF5 or any part thereof. There are also included antibodies recognizing only TRAF5 or any part thereof derived from a particular animal species such as human, and antibodies recognizing TRAF5 or any part thereof derived from two or more animal species.

The examples of the present antibodies are prepared using as an antigen the present TRAF5, polypeptide of each domain thereof, or fragments thereof. Thus, the DNA encoding the present TRAF5 is transformed into a suitable host cell to produce said TRAF5. The resulting TRAF5 is purified from the transformant or culture medium to use as an antigen for the production of the present antibodies in the method described later. It is also possible to synthesize chemically a polypeptide with a part of the amino acid sequence of the present TRAF5, and bind it to a carrier such as KLH (keyhole limpet hemocyanin) for use as an antigen for the production of the present antibodies in the method described later.

It is possible to prepare an antibody which recognizes TRAF5 with its whole length even using a part of the TRAF5 as an antigen. Also even if mouse TRAF5 or any part thereof is used as an antigen, an antibody which recognizes TRAF5 or any part thereof derived from human or other animal species than mouse may be prepared.

The present antibodies include monoclonal one and polyclonal one, which may be of any class or subclass. The present antibodies may be a chimera one or humanized one, or a fragment of the antibodies such as F(ab')2 and Fab, as long as they recognize TRAF5 or any part thereof.

The present antibodies may be prepared by the known method (e.g., "Meneki jikkenho (Laboratory manual of Immunology)" published by Japan Immunological Society), as exemplified below.

The DNA encoding the present TRAF5 is transformed into a suitable host cell to produce said TRAF5. The resulting TRAF5 is purified from the transformant or culture medium. Alternatively, the polypeptide with a part of the amino acid sequence of the present TRAF5 is synthesized chemically. These resulting TRAF5 and polypeptides are conjugated with a carrier such as KLH (keyhole limpet hemocyanin) and purified to obtain an antigen. The resulting antigen,alone or with a suitable adjuvant such as Freund's complete adjuvant (FCA) and Freund's incomplete adjuvant (FIA), is injected into animals at two to four-week intervals to immunize them. Blood is drawn from the immunized animals to obtain antiserum. The subject animals for immunization may be selected from rat, mouse, rabbit, sheep, horse, fowl, goat, pig, cattle and the like, depending on the type of an antibody to be desired. The polyclonal antibodies may be prepared by the purification of the resulting antiserum, using the known methods such as salting-out, ion-exchange chromatography, affinity chromatography and optional combination thereof.

Human antibodies may be prepared by in vitro sensitization method (Borrebaeck, C.A.K.J. Immunol., Meth., 123, 157, 1989), the method using SCID mouse (Toshio KUDO, Tissue Culture, 19, 61-65, 1993), etc.

The monoclonal antibodies may be prepared in the following way.

Antibody-producing cells such as spleen cells and lymphocytes are collected from the immunized animals, fused with myelomas and the like by known methods using polyethylene glycol, Sendai virus, electrical pulse to give hybridomas. Clones which produce the antibodies bonding to TRAF5 of the present invention are then selected and cultured. Monoclonal antibodies of the present invention are purified from the culture supernatant of the selected clones by known methods such as salting-out, ion-exchange chromatography, affinity chromatography and any combination thereof.

The chimera antibodies and humanized antibodies may be prepared by isolating the gene encoding the present antibodies from the hybridomas obtained above and utilizing it. For example, the chimera antibodies may be prepared by substituting a gene encoding the constant region of human antibodies for a gene encoding the constant region of the mouse antibodies, and expressing the thus reconstituted gene in animal cells. The humanized antibodies may be prepared by reconstituting a gene so that complementary determining regions (CDR) of the human antibodies are replaced with those of the mouse antibodies, and expressing the gene in animal cells (Carte et al., Pro. Nat. Acad. Sci, 89, 4285, 1992).

The present antibodies may be neutralizing antibodies, which inhibit the TRAF5 transduction of CD40-mediated signals. The neutralizing antibodies of the present invention include those that can completely inhibit the activity of TRAF5, and those partially inhibit the same.

The present antibodies may be labelled with fluorescent substances, enzymes, luminescent substances or radioisotopes to detect TRAF5 or their decomposed products present in body fluid or tissues. Since it is considered that TRAF5 is involved in transduction of CD40-mediated signals as already mentioned in the above, the detection of the existence of TRAF5 in blood or tissues would make it possible to estimate the progress of diseases and prognosis, and to confirm the effects of treatments. The present antibodies may be also used to provide an antibody-affinity column for the purification of TRAF5, or to detect TRAF5 in a fraction during the course of its purification.

The neutralizing antibodies of the present invention may serve as an effective ingredient of a medical composition for treating various diseases such as autoimmune disease by inhibiting or regulating the transduction of CD40-mediated signals.

Further, the present neutralizing antibodies may serve as an effective ingredient of a medical composition for the treatment of allergy by regulating the transduction of CD40-mediated signals to inhibit the exasperation of the production of IgE.

Also, the present invention relates to a vector comprising the DNA of the present invention. The present vector may further contain, if necessary, an enhancer sequence, promoter sequence, ribosome-binding sequence, base sequence for amplification of the number of copies, sequence encoding signal peptides, sequences encoding other polypeptides, poly(A)-additional sequence, splicing sequence, origin of replication, base sequence of the gene for selective markers and so on.

The present vector may be prepared by inserting the DNAs of the present TRAF5 or any part thereof into any vector according to the known methods (e.g., Sambrook J. et al., Molecular Cloning, a Laboratory Manual 2nd ed., Cold Spring Harbor Laboratory, New York, 1989). The preferable examples of the DNAs encoding TRAF5 or any part thereof are the base sequences shown as the SEQ ID No.2 or No.5 in the sequence Listing, or any part thereof. The present vectors include a plasmid vector, phage vector and virus vector such as pUC118, pBR322, pSV2-dhfr, pBluescriptII, pHIL-S1, λZapII, λgt10, pAc700, YRP17, pEF-BOS and pEFN-II.

The preferred vectors of the present invention may optionally comprise a promoter for expression in addition to the DNAs encoding TRAF5 or any part thereof to express TRAF5 or any part thereof.

The present expression vector maybe used to produce TRAF5 or any part thereof by means of genetic engineering.

The present invention therefore relates to a transformant by the above vectors. The present transformants may be prepared by transforming suitable host cells by the above vectors according to the known methods (e.g., Idenshi Kogaku Handbook (Handbook of gene technology), extra edition of Jikkenigaku, Yodo, 1991)). The host cells may be selected from procaryotic ones such as *E.coli* and *Bacillus*, or eucaryotic cells such as yeast, insect cells, and animal ones. The preferred transformants of the present invention are those derived from *E.coli*, yeast or CHO cell as a host cell to express the present TRAF5 or any part thereof.

The present invention further relates to a method for the production of TRAF5 or the present polypeptides comprising any part thereof, comprising the step of culturing the above transformants.

In the present production method, the transformants of the present invention are cultured, optionally with amplification of the gene or expression-induction, if necessary, according to the known methods (e.g. Biseibutsu Jikkenho (Laboratory manual of microbiology), Tokyo Kagaku Dojin, 1992). The culture mixture, i.e., the cells and culture supernatant, is collected and optionally subjected to concentration, solubilization, dialysis, and various chromatography such as affinity chromatography using the present antibodies to purify TRAF5 or the present polypeptides comprising any part thereof.

In the present production method, the polypeptides of the present invention may be produced by the transformants as a fusion protein with other polypeptides. In such case, the fusion protein would be treated with chemicals such as cyanogen bromide or enzymes such as protease in a certain step in the purification process, so that the polypeptides of the present invention may be excised therefrom.

The present invention also relates to a method for the screening of the substances using the present TRAF5, the polypeptides comprising any part thereof or the present antibodies against them, which substances, for example, will bind to present TRAF5 or the polypeptides, or regulate their activity or expression.

The substances binding to TRAF5 or the polypeptides comprising any part thereof, or the substances inhibiting the association between TRAF5 or the polypeptides comprising any part thereof and CD40 or the polypeptides comprising any part thereof may be screened using TRAF5 or the polypeptides comprising any part thereof, or CD40 or the polypeptides comprising any part thereof. For example, a fusion protein of TRAF5 or the polypeptides comprising any part thereof and FLAG epitope, and a fusion protein of CD40 or the polypeptides comprising any part thereof and GST are prepared according to the known method (Ishida, T. et al., Pro. Nat. Acad. Sci., 93, p.9437, 1996). These fusion proteins are then mixed with subject substances to select the substance inhibiting the association between TRAF5 or the polypeptides comprising any part thereof and CD40 or the polypeptides comprising any part thereof according to the same known method (Ishida, T. et al.).

Further, the substances inhibiting the association between TRAF5 or the polypeptides comprising any part thereof and CD40 or the polypeptides comprising any part thereof may be screened utilizing the two-hybrid method. For example, an expression vector for the expression of a fusion protein of the intracellular domain of CD40 and the DNA-binding domain of bacterial repressor LexA is prepared according to the same known method (Ishida, T. et al.). And an expression vector for the expression of a fusion protein of TRAF5 or the polypeptides comprising any part thereof and yeast protein GAL4 is prepared. These expression vectors are transformed into yeast strain L40 (Vojtek, A.B. et al., Cell, 74, p.205, 1993) to prepare a transformant according to the same known method (Ishida, T. et al.). The resulting transformant is then mixed with subject substances, followed by the detection of histidine requirement or β-galactosidase activity in order to select the substances inhibiting the association between TRAF5 or the polypeptides comprising any part thereof and CD40 or the polypeptides comprising any part thereof according to the same known method (Ishida, T. et al.).

According to the above known method (Ishida, T. et al.), substances may be screened on the basis of NFkB activation by TRAF5. For example, an expression vector of TRAF5 and a reporter plasmid for the evaluation of NFkB activation by TRAF5 are transformed into a human Jurkat cell or human 293T cell. The subject substances are added together and the expression of the reporter gene is detected in order to select the substance regulating the NFkB activation by TRAF5 or the polypeptides comprising any part thereof.

Further, the substances regulating the expression of TRAF5 or the polypeptides comprising any part thereof may be screened. For example, the subject substances are added to B cells, and the expression of TRAF5 or the polypeptides comprising any part thereof is determined by using the present antibodies against the present TRAF5.

The substances binding to or regulating the activity of TRAF5 or the polypeptides comprising any part thereof may be screened using TRAF5 or the polypeptides comprising any part thereof by the following way.

Thus, TRAF5 or CD40 or the polypeptides comprising any part thereof is massively produced, purified and crystallized according to the known method (Crystallization of Nucleic Acids and Proteins, A Practical Approach, Edited by A. Ducruix and R. Giege, IRL Press at Oxford University Press, 1992).

X-ray analysis is then carried out according to the known method (Methods in Enzymology Vol.114, Diffraction Methods for Biological Macromolecules Part A, Edited by Harold W. Wyckoff, C.H.W. Hirs and Serge N. Timasheff, Academic Press, Inc. 1985) to reveal the three-dimensional structure of TRAF5 or the polypeptides comprising any part thereof, or that of their complex with CD40 or the polypeptides comprising any part thereof.

The three-dimensional structure thus revealed may be analyzed according to the known method (Methods in Enzymology Vol.115, Diffraction Methods for Biological Macromolecules Part B, Edited by Harold W. Wyckoff, C.H.W. Hirs and Serge N. Timasheff, Academic Press, Inc. 1985).

The analytical data about the above three-dimensional structure thus obtained may be used to screen or design the substances binding to TRAF5 or the polypeptides comprising any part thereof, the substances inhibiting their association with CD40 or the polypeptides comprising any part thereof, or the substances inhibiting their activity.

The present invention therefore relates to the new substances thus screened.

Such substances binding to, or regulating the activity of TRAF5 or the polypeptides comprising any part thereof may be therefore used as a medicament with cell growth-inhibiting activity, or as a medicament to treat various immune diseases such as autoimmune disease by regulating the transduction of CD40-mediated signals.

Further, the above substances may be used as a medicament to treat allergy by regulating the transduction of CD40-mediated signals to inhibit the exasperation of the production of IgE.

The effective ingredients of the present invention may be formed into their salts or be modified with pharmaceutically acceptable chemical agents, as long as they will never lose their essential activities. There may be exemplified as the salts those with inorganic acids such as hydrochloric acid, phosphoric acid, hydrobromic acid and sulfuric acid; those with organic acids such as maleic acid, succinic acid, malic acid and tartaric acid.

The medical compositions of the present invention include those administered by any route such as oral, endermic, intravenous, intramuscular, intraperitoneal, intracutaneous, and intraintestinal ones.

The present medical compositions may be formulated according to the known methods depending on the administration route, and may comprise pharmaceutically acceptable auxiliaries such as excipients, filling agents, thickeners, binders, humectants, disintegrators, surfactants, solubilizers, buffers, pain-relieving agents, preservatives and stabilizers. In the case of injections, for example, they may comprise stabilizers such as gelatin, human serum albumin (HSA) and polyethylene glycol; alcohols and saccharides such as D-mannitol, D-sorbitol, and glucose; and surfactants such as Polysorbate 80 (TM).

The present medical compositions may be administered in an amount of about 0.01 ∼ 100 mg/kg/day, preferably of about 0.1 ∼ 10 mg/kg/day, depending on the conditions or ages of patients, or administration routes. The period for the administration is not specifically limited. It may also be continuously administered by an intravenous drip, or administered by a single dose or doses at appropriate intervals.

Summarized Description of Drawings
Fig.1 illustrates three clones associating specifically with each domain of TRAF5 and the intracellular domain of CD40.
Fig.2 shows comparison of amino acid sequences between TRAF5 and CRAF1.
Fig.3 shows the result in electrophoresis of Northern blotting of TRAF5 mRNA in various tissues.
Fig.4 shows the amino acid sequence of the intracellular domain of CD40 (from "K" at 216 to "Q" at 277) and its mutants.
Fig.5 shows the results in SDS-polyacrylamide gel electrophoresis and in electrophoresis of Western blotting of immune complex of between TRAF5 and the fusion protein consisting of GST and the intracellular domain of CD40 or its mutants.
Fig.6 shows the signal transduction activity of TRAF5 and CRAF1 using Jurkat cells and 293T cells.
Fig.7 shows the result in electrophoresis of Western blotting using the transformants of mouse WEHI-231 B cells.
Fig.8 shows the result of the inhibiting activity of induction of CD23 expression using FACS.
Fig.9 shows the result in electrophoresis of Northern blotting of human TRAF5 mRNA in the human B lymphoma cell lines, Daudi and Raji.
Fig.10 shows the signal transduction activity of TRAF5 using 293T cells.

### Best Mode for carrying Out the Invention

The present invention will be illustrated by the following examples which show the best mode of the present invention. Those examples, however, should not be construed to limit the scope of the present invention by any way.

The abbreviations used in the following description are based on the conventional ones in the art.

The operations in the following examples were done mainly in accordance with Sambrook J. et al., Molecular Cloning, a Laboratory Manual 2nd ed., Cold Spring Harbor Laboratory, New York, 1989; E. Harlow, D. Lane et al., Antibodies, A Laboratory Manual, Cold Spring Harbor Laboratory; and the like.

### Example 1: Preparation of DNA encoding mouse TRAF5

### (1) Screening

In order to clone cDNA encoding a protein associating with the intracellular domain of mouse CD40, two-hybrid screening method was carried out. The two-hybrid screening method is a method for the detection of complex-forming activity between a two kinds of fusion proteins on the basis of activation of transcription in budding yeast cells.

A murine C57 Black Kaplan T lymphoma cell line V13 cDNA library, which had been synthesized using an expression vector pACT, was purchased from CLONTECH. The cDNA of this library could be expressed as a fusion protein with the activation domain of yeast protein GAL4.

On the other hand, an expression vector, which may express the intracellular domain of mouse CD40 as a fusion protein with the DNA-binding domain of a bacterial repressor, LexA, was constructed in the following way.

The DNA fragment encoding the intracellular domain of mouse CD40 (Torres, R.M. et al., J. Immunol., Vol.148, 620-626, 1992: from the amino acid 216 (Lys) to the amino acid 305 (Phe)) was prepared by PCR in the following steps. At first, "5'-GCGGATCCTCAAAAAGGTGGTCAAGAAACCAAAG-3'" was synthesized as a sense primer, and "5'-GCGTCGACTCAAAAGGTCAGCAAGCAGCCATC-3'" was synthesized as an antisense primer. These primers were then mixed with cDNA of mouse WEHI-231 B cells as a template, Taq polymerase and reaction reagents (TOYOBO CO., LTD.). The reaction cycle of at 95°C for 1 min, 55°C for 2 min, and 72°C for 3 min was repeated 30 times using a DNA thermal cycler (Perkin Elmer) so as to collect an amplified product around 280 bp. After the digestion with BamHI and SalI, the product was inserted into the BamHI and SalI restriction enzyme sites of a plasmid pBTM116 (Bartel, P.L. et al., in Cellular Interactions in Development: A Practical Approach, Hartley, D.A., ed.: p.153-179 , Oxford University Press, Oxford, 1993). The thus constructed plasmid was named "pBTM40cyt."

HIS3 and lacZ genes had been integrated into the genome of the yeast strain L40 (vojtek, A.B. et al., Cell, Vol.74, p.205-214, 1993). Upon the association between the LexA DNA-binding domain/the intracellular domain of CD40 fusion protein and the activation domain of GAL4/the expression product of the above cDNAs, the yeast strain L40 would be able to grow in the absence of histidine, and would be positive for the β-galactosidase activity.

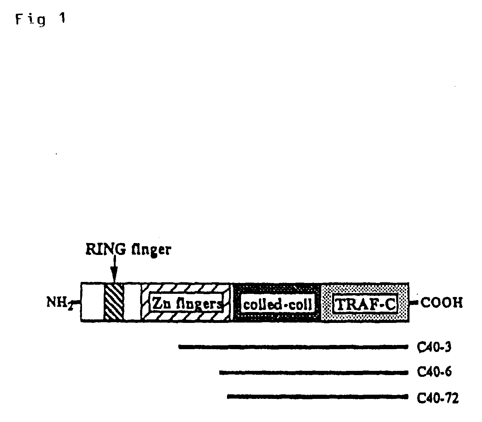

The pBTM40cyt was transformed into the yeast strain L40 by the lithium acetate method to give the transformant named "L40C40" expressing the LexA DNA-binding domain/the intracellular domain of CD40 fusion protein. 2 x 10⁶ clones of the above cDNA library were then transformed into the L40C40 by the lithium acetate method, and the resulting transformants were cultured in a histidine-free medium. After 7-day culture at 30°C, the grown clones were isolated and their β-galactosidase activity was detected in accordace with the protocol attached to the cDNA library. Seventy-two clones were selected, which showed detectable β-galactosidase activity within 20 min incubation. In order to remove cDNA clones of CRAF1 or TRAF2 which had been known to be selected by the same screening system, the selected clones were subjected to Southern blotting probed with CRAF1 or TRAF2 cDNA. Ten clones which did not hybridize with either of the two probes were used to collect the plasmids comprising the cDNA. The yeast strain L40 was cotransformed with the collected plasmids and pBTM40cyt or the vector (pBTMLamin) expressing the LexA DNA-binding domain/human lamin C fusion protein (Vojtek, A.B. et al., Cell, Vol.74, p.205-214, 1993) by the lithium acetate method. Four clones were selected, which could grow in the histidine-free medium, and showed β-galactosidase activity under the above condition only when they were cotransformed with the pBTM40cyt. Three clones (C40-3, C40-6, C40-72) of them were found to have cDNA encoding a part of the same protein (Fig.1).

The cDNA fragment of C40-3, which was the longest cDNA of the three clones, with about 1 kb was used as a probe to screen mouse testis cDNA library prepared by the known method in λZAPII vector (Stratagene) by the plaque hybridization method. Two independent clones were obtained, and the plasmids pBluescript having the same cDNA inserted therein were collected by in vivo excision method, followed by nucleotide sequencing with the BcaBest sequence system (Takara Shuzo). One of the two clones was revealed to comprise the longest cDNA fragment with 2105 bp (SEQ ID No. 3 in the Sequence Listing). The plasmid pBluescript into which the longest cDNA fragment had been inserted was named "pBSCRAF2 (pBSTRAF5)."

The pBSCRAF2 (pBSTRAF5) was transformed into E.coli strain NM522 by the known method, and the resulting E.coli NM522 transformant was deposited in the National Institute of Bioscience and Human-Technology, Agency of Industrial Science and Technology (1-3, Higashi 1-chome, Tsukuba-shi, Ibaraki-ken 350 Japan) on March 27, 1996 under accession numbers FERM P-15531, and then transferred on March 6, 1997 to the deposit under the terms of the Budapest Treaty on the International Recognition of the Deposit of Microorganisms for the Purposes of Patent Procedure and Regulation under accession numbers FERM BP-5856.

### (2) Analysis of the structure of TRAF5

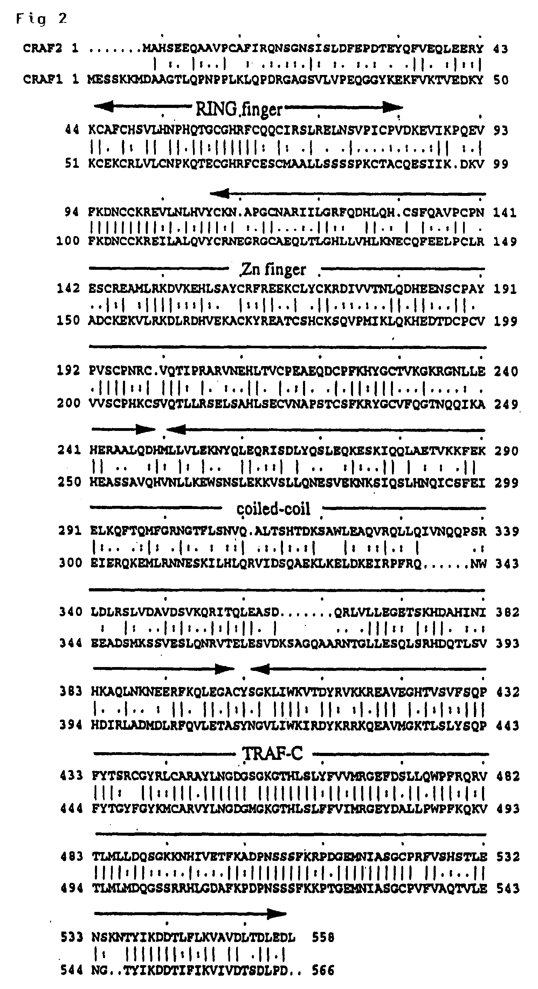

The analysis of the structure of TRAF5 based on the nucleotide sequence determined in the above suggested that TRAF5 was a protein consisting of 558 amino acid residues (SEQ ID No.1 in the Sequencing Listing). Homology searching against PIR data base showed its highest homology to CRAF1, as shown in Fig.2. Especially, it was revealed that a TRAF-C domain existed at the C-terminal region of TRAF5 (Fig.2). The TRAF-C domain is a motif which is known to be involved in the association with other proteins and to be present commonly in TRAF1 and TRAF2 which are known to associate with the intracellular domain of TNFR-2, and in CRAF1. It has been revealed that TRAF5 has a RING finger domain, five Zn finger domains and a coiled-coil domain in addition to the TRAF-C domain, in the order from N-terminus (Fig.1).

### (3) Northern blotting

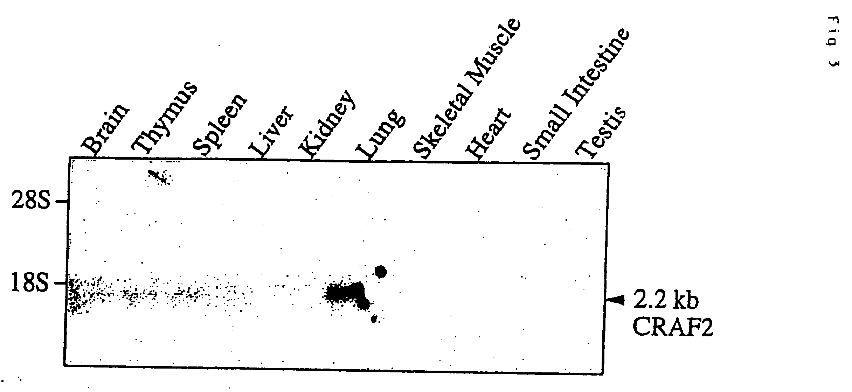

The total mRNA from various tissues was prepared by the guanidine isothiacyanate/acid-phenol method (Chomczynski,P. and Sacchi, N., Anal. Biochem., Vol.162, p.156-159, 1987), and poly(A)⁺RNA was purified using oligo(dT)latex (Takara Shuzo). Seven micrograms of poly(A)⁺RNA was subjected to electrophoresis on 1% agarose gel containing 6.6% formaldehyde and transferred to a nylon membrane filter (Amersham). The nylon membrane was incubated with the probe of ³²P-labeled C40-3 cDNA fragment in hybridization buffer (0.2 M NaHPO₄(pH 7.2), 1mM EDTA, 1%(w/v) BSA, 7%(w/v) SDS) at 65°C. The filter was finally washed with 0.5 x SSC/0.2%(w/v) SDS at 65°C for 30 min, followed by autoradiography. The result is shown in Fig.3.

The TRAF5 mRNA was highly expressed in lung, moderately expressed in thymus, spleen and kidney, and weakly expressed in brain and liver. However, TRAF5 mRNA was not detected by Northern blotting in skeletal muscle, heart, small intestine and testis. The detection of TRAF5 mRNA with about 2.2kb confirmed that the resulting TRAF5 cDNA was a full-length copy of the corresponding mRNA.

### Example 2: Determination of human CD40 region necessary for the association with TRAF5

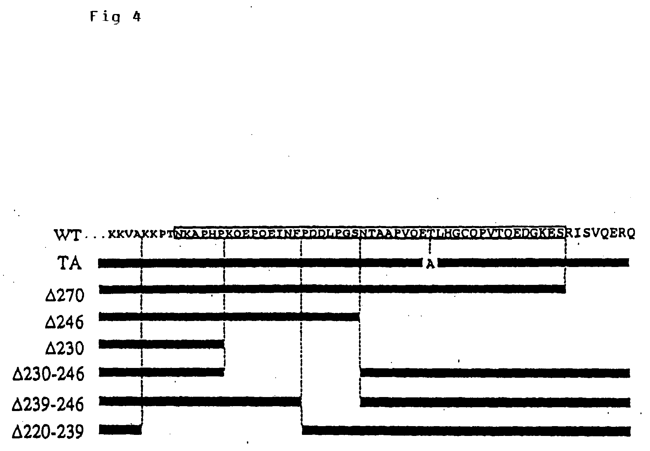

Plasmids encoding mutants of the intracellular domain of CD40 (Stamenkovic, I. et al., EMBO J., Vol.8, p.1403-1410, 1989; Fig.4) were prepared in accordance with the method of Kunkel (Kunkel, T. A., Proc. Natl. Acad. Sci. USA, Vol.82, p.488-492, 1985). The DNAs encoding human CD40, its mutants, or the intracellular domain of human TNFR-2 (Smith, C.A. et al., Science, Vol.248, p.1019-1023, 1990: from amino acid 288 (Lys) to amino acid 461 (Ser)), were subcloned into the GST fusion protein expression vector pGEX2T (Pharmacia LKB), respectively, and transformed into the E. coli strain BL21. The mutation sites in the intracellular domain of human CD40, which were encoded by the expression vectors, are shown in Fig. 4.

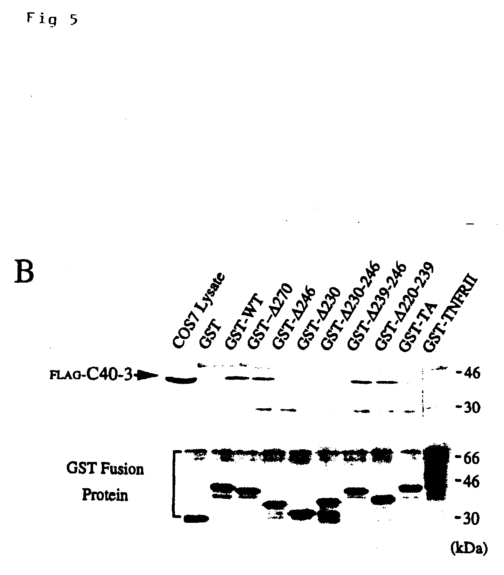

GST, GST/the intracellular domain of CD40 or its mutants fusion protein, and GST/TNFR-2 fusion protein (GST-TNFR II) were prepared in accordance with the method of Smith et al (Smith, D.B. and Johnson, K.S., Gene, Vol.67, p.31-40, 1988), and the resulting proteins were immobilized onto glutathione-agarose beads at a concentration of 0.2 mg/ml. Two µl of each bead solution was subjected to electrophoresis on 12.5 % polyacrylamide/SDS gel and stained with Coomassie Brilliant Blue R-250. The results were shown in the lower part of Fig. 5.

The expression vector pME-FLAG-C40-3 was prepared by inserting the DNA encoding the protein encoded by the C40-3 cDNA and tagged with FLAG epitope (Eastman Kodak) at its amino terminus into downstream of SRα promoter of the expression vector pME18S (Bio Mannual Series 4, Gene transfection and Expression, Analytical Method, Extra Edition of Jikkenigaku, Yodo, published April 20, 1994).

Ten micrograms of pME-FLAG-C40-3 were transfected into 10⁶ of COS7 cells. The transfected cells were harvested 36 hr after the transfection, lysed with TNE buffer(10 mM Tris-HCL (pH 7.8), 1%(W/V) NP-40, 0. 15M NaCl, 10mM iodoacetoamide, 1mM EDTA, 10µg/ml aprotinin) and centrifuged. One-half of the lysate was incubated with 1 µg of the above proteins immobilized onto glutathione-agarose beads at 4°C for one hour. The beads were washed and boiled in the presence of 0.1% SDS followed by immune precipitation using anti-FLAG antibody M2 (Eastman Kodack). The immune complexes were subjected to electrophoresis on 12.5% polyacrylamide/SDS gel. Western blotting was then carried out using anti-FLAG antibody M2 and anti-mouse IgG antibody labeled with alkaline phosphatase by the known method. The results are shown in the upper part of Fig.5.

GST/the intracellular domain of CD40 fusion protein (GST-WT) associated well with FLAG-C40-3. The specificity of the binding (association) in this experiment was confirmed by the fact that the GST protein used as a negative control did not associate with FLAG-C40-3. On the other hand, the binding activity with FLAG-C40-3 of the mutant (GST-TA: Fig.4) was significantly reduced in comparison with GST-WT, wherein Thr-254 had been replaced by Ala. It was already known that such alternation would disable CD40 signaling linked to growth inhibition (Inui, S. et al., Eur. J. Immunol., Vol.20, p.1747-1753, 1990). Among other CD40 mutants with the deletion in its intracellular domain, GST-Δ270 (deletion of the amino acid residues 270 (Arg) - 277 (Gln) in Fig.4) showed almost the same binding activity as GST-WT, but GST-Δ230 (deletion of the amino acid residues 230 (Lys) - 277 (Gln)) and GST-Δ246 (deletion of the amino acid residues 246 (Asn) - 277 (Gln)) could hardly associate with FLAG-C40-3. on the other hand, compared with GST-Δ230 and GST-Δ246, GST-Δ230-2A6 (deletion of the amino acid residues 230 (Asn) - 245 (Ser)) associated with FLAG-CA0-3 a little. GST-Δ239-246 (deletion of the amino acid residues 239 (Pro) - 245 (Ser)) and GST-Δ220-239 (deletion of the amino acid residues 220 (Lys) - 238 (Phe)) also showed almost the same binding activity as GST-WT.

From the above results, it has been found that the region between 246 (Asn) and 269 (Ser) is neccesary but enough for the association with TRAF5, and that either the region between 230 (Lys) and 239 (Pro) or the region between 239 (Pro) and 246 (Asn) is additionally required for the efficient association with TRAF5. Although the intracellular domain of CD40 has not yet analyzed with respect to its steric structure, is seems that TRAF5 will recognize the region ranging from 230 (Lys) to 269 (Ser) of the structure of CD40.

Incidentally, it has been reported that CRAF1 associates slightly with TNFR-2 (Mosialos, G., et al., Cell, Vol.80, p.389-399, 1995). On the other hand, GST-TNFRII(TNFR-2) did not associate with FLAG-C40-3, as shown in the upper part of Fig.5, indicating that TRAF5 would not associate with TNFR-2.

### Example 3: Confirmation of the signal transduction activity of TRAF5

### (1) Confirmation of activation of NFκB

Human Jurkat T cells were cultured in RPMI 1640 medium supplemented with 10% fetal bovine serum. Human 293T kidney cells were cultured in DME medium supplemented with 10% fetal bovine serum.

CRAF1 cDNA was prepared by PCR in the following steps. At first, "5'-CTCCTCGAGATGGAGTCGAGTAAAAAGATGGAC-3'" was synthesized as a sense primer, and "5'-CTTACTAGTTCAGGGATCGGGCAGATCCGAAGT-3'" was synthesized as an antisense primer. These primers were then mixed with cDNA of mouse spleen as a template, Taq polymerase and reaction reagents (TOYOBO CO., LTD.). The reaction cycle of at 95°C for 1 min, 55°C for 2 min, and 72°C for 3 min was repeated 30 times using a DNA thermal cycler (Perkin Elmer) so as to collect an amplified product around 1500 bp. After the digestion with XhoI and SpeI, the product was inserted into the XhoI and SpeI restriction enzyme sites of an expression vector pME18S. The thus constructed plasmid was named "pME-CRAF1." on the other hand, TRAF5 cDNA was inserted into the EcoRI and NotI restriction enzyme sites of an expression vector pME18S. The thus constructed plasmid was named "pME-TRAF5 (pME-CRAF2)."

In order to evaluate the activity of transcription factor NE-κB, [κB]₆TK-CAT was used as a reporter plasmid, wherein CAT would be expressed depending on a κB site as an NF-κB binding site (Inoue, J., et al., Proc. Natl. Acad. Sci. USA., Vol.88, p.3715-3719, 1991). Further, to confirm the κB specificity of CAT expression, [κBM]₆TK-CAT was used as a negative control reporter plasmid, wherein κB site had been mutated (Inoue, J., et al., Proc. Natl. Acad. Sci. USA., Vol.88, p.3715-3719, 1991). β-actin-β-gal expressing β-galactosidase driven by β-actin promoter was also used as a reporter plasmid to evaluate the DNA transfection efficiency into cells.

The transfection of the expression vectors into Human Jurkat T cells was carried out in the following way.

One microgram of the reporter plasmid ( [κB]₆TK-CAT or [κBM]₆TK-CAT ), 1 µg of β-actin-β-gal and 1.5 µg or 3 µg of pME-CRAF1 or pME-TRAF5 were mixed together, followed by the addition of pME18S to a total DNA amount of 5 µg. The mixed DNAs were cotransfected into Jurkat T cells of 2x10⁶ by the DEAE-dextran method.

The transfection of the expression vectors into Human 293T kidney cells was carried out in the following way.

One microgram of the reporter plasmid ( [κB]₆TK-CAT or [κBM]₆TK-CAT ), 1 µg of β-actin-β-gal and 10 µg or 20 µg of pME-CRAF1 or pME-TRAF5 were mixed together, followed by the addition of pME18S to a total DNA amount of 22 µg. The mixed DNAs were cotransfected into Human 293T kidney cells of 10⁶ by the calcium phosphate method.

Forty-eight hours after transfection, cell extracts were prepared by collecting the cells, followed by freeze-thawing and centrifugation.

β-galactosidase activity was determined to standardize the transfection efficiency according to the method (Herbomel, P., et al., Cell, Vol.39, p.653-662, 1984).

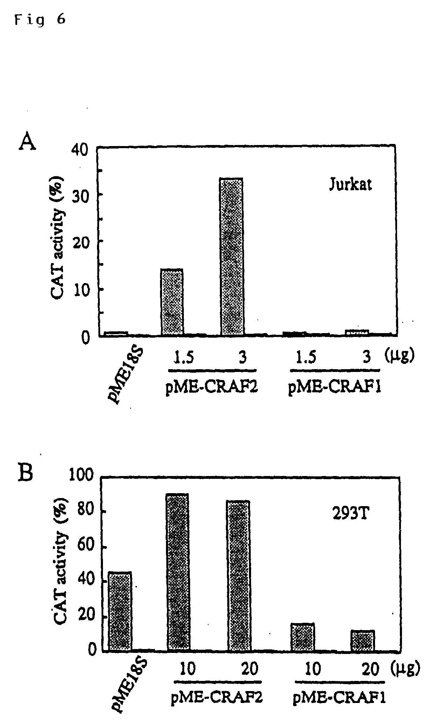

CAT activity was determined at 37°C for 1 hr according to the method (Gorman, C.M., et al., Mol. Cell. Biol., Vol.2, p.1044-1051, 1982). The results are shown in Fig. 6.

TRAF5 activated the κB site-dependent transcription in human Jurkat T cells (A) in a dose-dependent manner. But CRAF1 did not show such activity. Although TRAF5 activated NFκB activation also in human 293T kidney cells (B), but its dose-dependency was not so significant as seen in human Jurkat T cells. It was because NFκB had been already activated to some extent without stimulation in human 293T kidney cells. This pre-activated NFκB activity was suppressed by the overexpression of CRAF1, indicating that TRAF5 and CRAF1 showed conflicting activities with each other with respect to the activation of NFκB by their overexpression.

### (2) Confirmation of the dominant-negative mutant 's inhibiting activity of the induction of CD23 expression

Mouse WEHI-231 B cells were cotransfected with pME-FLAG-C40-3 and an expresson vector (pApuro) for the puromycin resistant gene (Takata, M. et al., EMBO J., Vol.13, p.1341-1349, 1994), followed by the selection in the presence of 0.5µg/ml of puromycin to obtain the transformants.

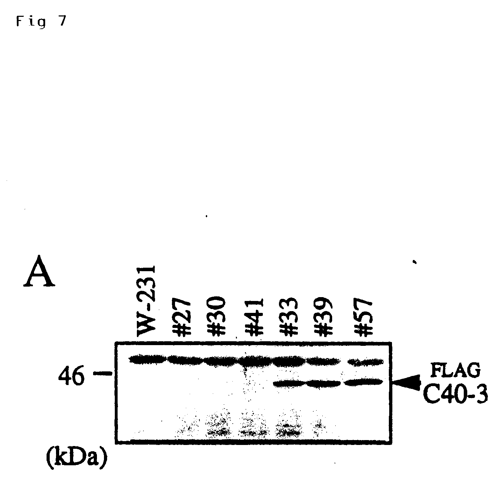

The expression of FLAG-C40-3 was checked for #27, #30, #41, #33, #39, #57 and their parent cell line, WEHI-231 B cells by the Western blotting method of Example 2. The clones of #33, #39 and #57 were confirmed to express FLAG-C40-3 (Fig.7). On the other hand, it was not confirmed that the clones of #27, #30, #41, and WEHI-231 B cells expressed the same protein (Fig.7). All of the transformants were confirmed to express normal levels of mouse CD40.

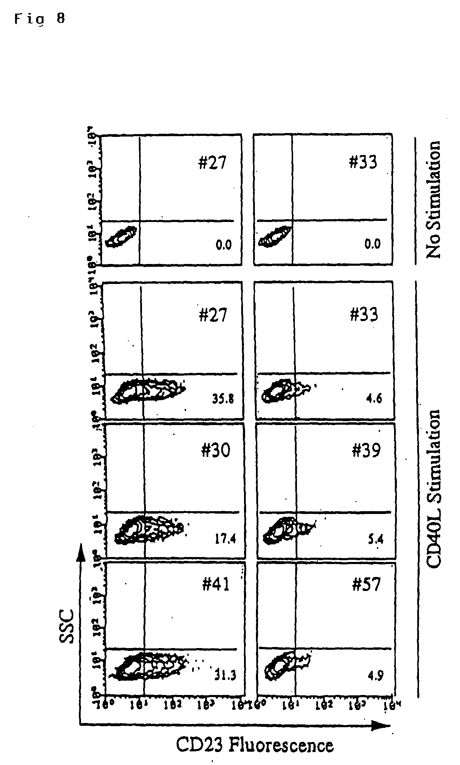

The above transformants were stimulated with mouse CD40L-CD8 chimeric protein (Lane, P., et al., J Exp. Med., vol.177, p.1209-1213, 1993) for 48 hr. For non-stimulating control, medium was added to instead of the stimulator. The transformant cells were then stained with fluorescein isothiocyanate-conjugated anti-CD23 antibody followed by FACScan (Becton Dickinson) analysis using the Lysis II program. The results are shown in Fig.8.

Induction of CD23 expression was scarcely observed in #33, #39 and #57, while the parent cells and #27, #30, #41 expressed CD23 after the stimulation by the CD40L-CD8 chimeric sitmulator. The protein encoded by the cDNA of C40-3 lacks in the N-terminus region of TRAF5, and does not have RING finger domain and nor part of Zn finger domain, but does have TRAF-C domain (Fig.1). It was revealed that this protein acted as a dominant negative mutant for the CD40-mediated induction of CD23 expression.

### Example 4: Preparation of DNA encoding human TRAF5

### (1) Screening

The cDNA library of Burkit B lymphoma cell line, Daudi (Clontech) was screened using the cDNA fragment of mouse TRAF5 obtained in Example 1 by the Plague hybridization method. The hybridisation was carried out by incubation in the hybridisation buffer (0.2 M NaHPO₄(pH 7.2), 1mN EDTA, 1%(w/v) BSA, 7%(w/v) SDS) at 50°C. The filter was finally washed with 1 x SSC/0.1%(w/v) SDS at 50°C for 30 min, followed by autoradiography. Two independent clones were obtained, and their cDNA fragments were subcloned into a plasmid pBluescript, followed by nucleotide sequencing with the ABI PRIZM cycle sequence system (Perkin Elmer). One clone was revealed to comprise the longest cDNA fragment with 3993 bp (SEQ ID No.6 in the Sequence Listing). The plasmid psluescript into which the longest cDNA fragment had been inserted was named "pBShTRAF5."

The pBShTRAF5 was transformed into E.coli strain JM109 by the known method, and the resulting E.coli JM109 transformant was deposited in the National Institute of Bioscience and Human-Technology, Agency of Industrial Science and Technology (1-3, Higashi 1-chome, Tsukubashi, Ibaraki-ken 350 Japan) on December 10, 1996 under accession numbers FERM P-15993, and then transferred on March 6, 1997 to the deposit under the terms of the Budapest Treaty on the International Recognition of the Deposit of Microorganisms for the Purposes of Patent Procedure and Regulation under accession numbers FERN BP-5857.

### (2) Analysis of the structure of human TRAF5

The analysis of the structure of human TRAF5 based on the nucleotide sequence determined in the above (1) suggested that human TRAF5 was a protein consisting of 557 amino acid residues (SEQ ID No.4 in the Sequencing Listing). It has been revealed that human TRAF5 has 80% homology in amino acid level and 82% homology in DNA nucleotide level to mouse TRAF5. It has a RING finger domain, five Zn finger domains, a coiled-coil domain and TRAF-C domain in the order from its N-terminus.

### (3) Northern blotting

Poly(A)⁺RNA of Human B lymphoma cell lines, Daudi and Raji were prepared by the same way as Example 1. Poly(A)⁺RNA (12µg) was subjected to electrophoresis on 1% agarose gel containing 6.6% hormaldehyde and transferred to a nylon membrane (Amersham). Probes were prepared as follows.

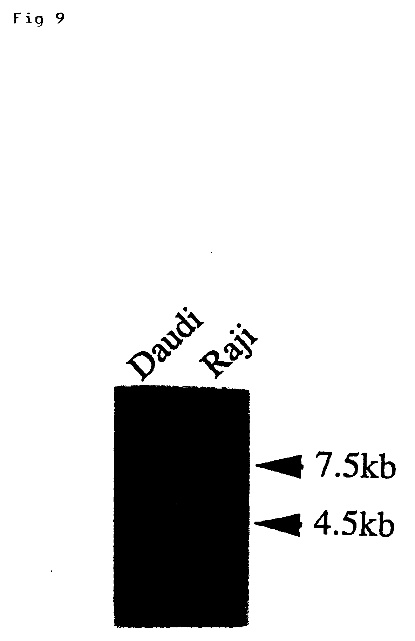

At first, "5'-GCAGCAGCCGCGCCTGCAGACCGGC-3'" was synthesized as a sense primer, and "5'-ATCCAGGAGCATTGCTGCAATATAC-3'" was synthesized as an antisense primer. These primers were then mixed with human TRAF5 cDNA as a template, Taq polymerase and reaction reagents (TOYOBO CO., LTD.). The reaction cycle of at 95°C for 1 min, 55°C for 2 min, and 72°C for 3 min was repeated 30 times using a DNA thermal cycler (Perkin Elmer) so as to collect an amplified product around 500 bp. The resulting DNA fragment was labelled with ³²P. The nylon membrane was incubated with the ³²P-labeled probe in hybridization buffer (0.2 M NaHPO₄(pH 7.2), 1mM EDTA, 1%(w/v) BSA, 7%(w/v) SDS) at 65°C. The filter was finally washed with 0.5 x SSC/0.2%(w/v) SDS at 65°C for 30 min, followed by autoradiography. The result is shown in Fig.9.

The size of the detected human TRAF5 mRNA was about 4∼5 kb, confirming that the resulting human TRAF5 cDNA was almost a full-length copy of the corresponding mRNA.

### Example 5: Confirmation of signal trannsduction activity of

### (1) Confirmation of activation of NFκB

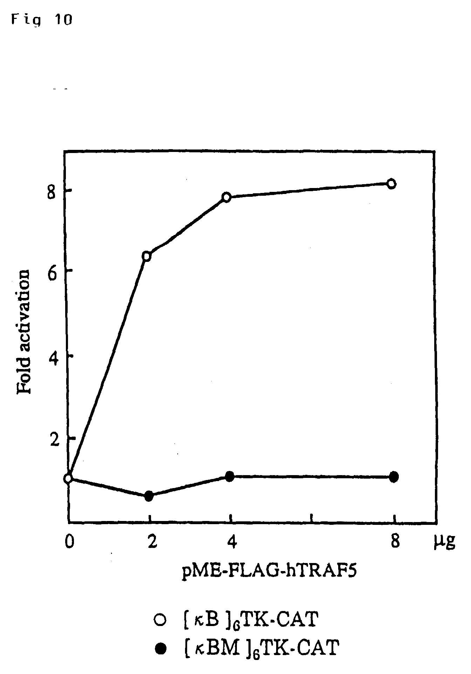

Human TRAF5's function of activating NFκB was confirmed by the same method as in Example 3. One microgram of the reporter plasmid ( [κB]₆TK-CAT or [κBM]₆TK-CAT ), 1 µg of β-actin-β-gal and 2, 4 or 8 µg of pME-FLAG-hTRAF5 were mixed together, followed by the addition of pME18S to a total DNA amount of 10 µg. No pME-FLAG-hTRAF5 was added to a sample used as a negative control. The mixed DNAs were cotransfected into 293T cells of 2 x 10⁶ by the calcium phosphate method. Forty eight hours after transfection, cell extracts were prepared by collecting the cells, followed by freeze-thawing and centrifugation. CAT activity was determined. The results are shown in Fig.10.

Human TRAF5 activated the κB site-dependent transcription in 293T T cells in a dose-dependent manner.

SEQUENCE LISTING

SEQ ID NO : 1
Length : 558
Type : amino acid
Topology : linear
MOLECULE TYPE : peptide
ORIGINAL SOURCE
  ORGANISM : mouse
SEQUENCE DESCRIPTION

Met Ala His Ser Glu Glu Gln Ala Ala Val Pro Cys Ala Phe Ile Arg
  1               5                  10                  15

Gln Asn Ser Gly Asn Ser Ile Ser Leu Asp Phe Glu Pro Asp Thr Glu
             20                  25                  30

Tyr Gln Phe Val Glu Gln Leu Glu Glu Arg Tyr Lys Cys Ala Phe Cys
         35                  40                  45

His Ser Val Leu His Asn Pro His Gln Thr Gly Cys Gly His Arg Phe
     50                  55                  60

Cys Gln Gln Cys Ile Arg Ser Leu Arg Glu Leu Asn Ser Val Pro Ile
 65                  70                  75                  80

Cys Pro Val Asp Lys Glu Val Ile Lys Pro Gln Glu Val Phe Lys Asp
                 85                  90                  95

Asn Cys Cys Lys Arg Glu Val Leu Asn Leu His Val Tyr Cys Lys Asn
            100                 105                 110

Ala Pro Gly Cys Asn Ala Arg Ile Ile Leu Gly Arg Phe Gln Asp His
        115                 120                 125

Leu Gln His Cys Ser Phe Gln Ala Val Pro Cys Pro Asn Glu Ser Cys
    130                 135                 140

Arg Glu Ala Met Leu Arg Lys Asp Val Lys Glu His Leu Ser Ala Tyr
145                 150                 155                 160

Cys Arg Phe Arg Glu Glu Lys Cys Leu Tyr Cys Lys Arg Asp Ile Val
                165                 170                 175

Val Thr Asn Leu Gln Asp His Glu Glu Asn Ser Cys Pro Ala Tyr Pro
            180                 185                 190

Val Ser Cys Pro Asn Arg Cys Val Gln Thr Ile Pro Arg Ala Arg Val
        195                 200                 205

Asn Glu His Leu Thr Val Cys Pro Glu Ala Glu Gln Asp Cys Pro Phe
    210                 215                 220

Lys His Tyr Gly Cys Thr Val Lys Gly Lys Arg Gly Asn Leu Leu Glu
225                 230                 235                 240

His Glu Arg Ala Ala Leu Gln Asp His Met Leu Leu Val Leu Glu Lys
                245                 250                 255

Asn Tyr Gln Leu Glu Gln Arg Ile Ser Asp Leu Tyr Gln Ser Leu Glu
            260                 265                 270

Gln Lys Glu Ser Lys Ile Gln Gln Leu Ala Glu Thr Val Lys Lys Phe
        275                 280                 285

Glu Lys Glu Leu Lys Gln Phe Thr Gln Met Phe Gly Arg Asn Gly Thr
    290                 295                 300

Phe Leu Ser Asn Val Gln Ala Leu Thr Ser His Thr Asp Lys Ser Ala
305                 310                 315                 320

Trp Leu Glu Ala Gln Val Arg Gln Leu Leu Gln Ile Val Asn Gln Gln
                325                 330                 335

Pro Ser Arg Leu Asp Leu Arg Ser Leu Val Asp Ala Val Asp Ser Val
            340                 345                 350

Lys Gln Arg Ile Thr Gln Leu Glu Ala Ser Asp Gln Arg Leu Val Leu
        355                 360                 365

Leu Glu Gly Glu Thr Ser Lys His Asp Ala His Ile Asn Ile His Lys
    370                 375                 380

Ala Gln Leu Asn Lys Asn Glu Glu Arg Phe Lys Gln Leu Glu Gly Ala
385                 390                 395                 400

Cys Tyr Ser Gly Lys Leu Ile Trp Lys Val Thr Asp Tyr Arg Val Lys
                405                 410                 415

Lys Arg Glu Ala Val Glu Gly His Thr Val Ser Val Phe Ser Gln Pro
            420                 425                 430

Phe Tyr Thr Ser Arg Cys Gly Tyr Arg Leu Cys Ala Arg Ala Tyr Leu
        435                 440                 445

Asn Gly Asp Gly Ser Gly Lys Gly Thr His Leu Ser Leu Tyr Phe Val
    450                 455                 460

Val Met Arg Gly Glu Phe Asp Ser Leu Leu Gln Trp Pro Phe Arg Gln
465                 470                 475                 480

Arg Val Thr Leu Met Leu Leu Asp Gln Ser Gly Lys Lys Asn His Ile
                485                 490                 495

Val Glu Thr Phe Lys Ala Asp Pro Asn Ser Ser Ser Phe Lys Arg Pro
            500                 505                 510

Asp Gly Glu Met Asn Ile Ala Ser Gly Cys Pro Arg Phe Val Ser His
        515                 520                 525

Ser Thr Leu Glu Asn Ser Lys Asn Thr Tyr Ile Lys Asp Asp Thr Leu
    530                 535                 540

Phe Leu Lys Val Ala Val Asp Leu Thr Asp Leu Glu Asp Leu
545                 550                 555

SEQ ID NO : 2
LENGTH : 1674
TYPE : nucleic acid
STRANDNESS : double
TOPOLOGY : linear
MOLECULE TYPE : cDNA to mRNA
ORIGINAL SOURCE
  ORGANISM : mouse
FEATURE
  Feature Key : CDS
  Location : 1..1674
  Method for the determination of feature : P
SEQUENCE DESCRIPTION

ATG GCT CAT TCG GAG GAG CAA GCG GCT GTC CCC TGC GCC TTC ATC CGC        48
Met Ala His Ser Glu Glu Gln Ala Ala Val Pro Cys Ala Phe Ile Arg
  1               5                  10                  15

CAG AAC TCT GGC AAC TCA ATT TCC TTG GAC TTT GAG CCC GAC ACC GAG 96
Gln Asn Ser Gly Asn Ser Ile Ser Leu Asp Phe Glu Pro Asp Thr Glu
            20                  25                  30

TAC CAG TTT GTG GAG CAG CTG GAA GAA CGC TAC AAA TGT GCC TTC TGC 144
Tyr Gln Phe Val Glu Gln Leu Glu Glu Arg Tyr Lys Cys Ala Phe Cys
        35                  40                  45

CAC TCC GTG CTT CAC AAC CCC CAC CAG ACC GGC TGC GGG CAC CGC TTC 192
His Ser Val Leu His Asn Pro His Gln Thr Gly Cys Gly His Arg Phe
    50                  55                  60

TGC CAG CAG TGC ATC CGG TCT CTG AGA GAA TTG AAC TCG GTG CCG ATC 240
Cys Gln Gln Cys Ile Arg Ser Leu Arg Glu Leu Asn Ser Val Pro Ile
65                  70                  75                  80

TGC CCG GTA GAC AAG GAG GTC ATC AAG CCT CAG GAG GTG TTC AAA GAC 288
Cys Pro Val Asp Lys Glu Val Ile Lys Pro Gln Glu Val Phe Lys Asp
                85                  90                  95

AAC TGC TGC AAA AGA GAA GTT CTC AAT TTA CAC GTC TAC TGC AAA AAC 336
Asn Cys Cys Lys Arg Glu Val Leu Asn Leu His Val Tyr Cys Lys Asn
            100                 105                 110

GCC CCC GGG TGC AAT GCC AGG ATT ATT CTG GGA CGA TTC CAG GAC CAC 384
Ala Pro Gly Cys Asn Ala Arg Ile Ile Leu Gly Arg Phe Gln Asp His
        115                 120                 125

CTT CAG CAC TGT TCC TTC CAA GCC GTG CCC TGC CCT AAC GAG AGC TGC 432
Leu Gln His Cys Ser Phe Gln Ala Val Pro Cys Pro Asn Glu Ser Cys
    130                 135                 140

CGG GAA GCC ATG CTC CGG AAA GAC GTG AAA GAG CAC CTG AGC GCA TAC 480
Arg Glu Ala Met Leu Arg Lys Asp Val Lys Glu His Leu Ser Ala Tyr
145                 150                 155                 160

TGC CGG TTC CGA GAG GAG AAG TGC CTT TAC TGC AAA AGG GAC ATA GTG 528
Cys Arg Phe Arg Glu Glu Lys Cys Leu Tyr Cys Lys Arg Asp Ile Val
                165                 170                 175

GTG ACC AAC CTG CAG GAT CAT GAG GAA AAC TCG TGT CCT GCG TAC CCA 576
Val Thr Asn Leu Gln Asp His Glu Glu Asn Ser Cys Pro Ala Tyr Pro
            180                 185                 190

GTG TCT TGT CCC AAC AGG TGT GTG CAG ACT ATT CCA AGA GCT AGG GTG 624
Val Ser Cys Pro Asn Arg Cys Val Gln Thr Ile Pro Arg Ala Arg Val
        195                 200                 205

AAT GAA CAC CTT ACT GTA TGT CCT GAG GCT GAG CAA GAC TGT CCC TTT 672
Asn Glu His Leu Thr Val Cys Pro Glu Ala Glu Gln Asp Cys Pro Phe
    210                 215                 220

AAG CAC TAT GGC TGC ACT GTC AAG GGT AAG CGG GGG AAC TTG CTG GAG 720
Lys His Tyr Gly Cys Thr Val Lys Gly Lys Arg Gly Asn Leu Leu Glu
225                 230                 235                 240

CAT GAG CGG GCA GCC CTG CAG GAC CAC ATG CTT CTG GTT TTA GAG AAG 768
His Glu Arg Ala Ala Leu Gln Asp His Met Leu Leu Val Leu Glu Lys
                245                 250                 255

AAC TAC CAA CTA GAA CAG CGG ATC TCT GAT TTA TAT CAG AGT CTC GAA 816
Asn Tyr Gln Leu Glu Gln Arg Ile Ser Asp Leu Tyr Gln Ser Leu Glu
            260                 265                 270

CAG AAG GAA AGC AAG ATC CAG CAG CTG GCA GAA ACC GTG AAG AAG TTC 864
Gln Lys Glu Ser Lys Ile Gln Gln Leu Ala Glu Thr Val Lys Lys Phe
        275                 280                 285

GAA AAG GAG CTT AAG CAG TTC ACA CAG ATG TTT GGC AGA AAT GGA ACT 912
Glu Lys Glu Leu Lys Gln Phe Thr Gln Met Phe Gly Arg Asn Gly Thr
    290                 295                 300

TTC CTC TCA AAT GTC CAG GCT CTC ACC AGT CAC ACG GAC AAG TCA GCT 960
Phe Leu Ser Asn Val Gln Ala Leu Thr Ser His Thr Asp Lys Ser Ala
305                 310                 315                 320

TGG CTG GAA GCG CAG GTG CGG CAG CTG CTA CAA ATA GTT AAC CAG CAG 1008
Trp Leu Glu Ala Gln Val Arg Gln Leu Leu Gln Ile Val Asn Gln Gln
                325                 330                 335

CCA AGT CGA CTT GAT CTG AGG TCT TTG GTG GAT GCG GTT GAC AGC GTG 1056
Pro Ser Arg Leu Asp Leu Arg Ser Leu Val Asp Ala Val Asp Ser Val
            340                 345                 350

AAA CAG AGG ATC ACC CAG CTG GAA GCC AGT GAC CAG AGA TTA GTT CTT 1104
Lys Gln Arg Ile Thr Gln Leu Glu Ala Ser Asp Gln Arg Leu Val Leu
        355                 360                 365

TTA GAG GGG GAG ACC AGC AAG CAC GAC GCA CAC ATT AAT ATC CAC AAA 1152
Leu Glu Gly Glu Thr Ser Lys His Asp Ala His Ile Asn Ile His Lys
    370                 375                 380

GCA CAG CTG AAT AAG AAC GAA GAG CGG TTT AAG CAG CTG GAG GGC GCC 1200
Ala Gln Leu Asn Lys Asn Glu Glu Arg Phe Lys Gln Leu Glu Gly Ala
385                 390                 395                 400

TGC TAC AGT GGC AAG CTC ATC TGG AAG GTG ACA GAT TAC AGG GTG AAG 1248
Cys Tyr Ser Gly Lys Leu Ile Trp Lys Val Thr Asp Tyr Arg Val Lys
                405                 410                 415

AAG AGG GAG GCC GTG GAG GGG CAC ACA GTG TCC GTC TTC AGC CAG CCT 1296
Lys Arg Glu Ala Val Glu Gly His Thr Val Ser Val Phe Ser Gln Pro
            420                 425                 430

TTC TAC ACC AGC CGC TGC GGC TAC CGG CTC TGT GCC AGG GCG TAC CTG 1344
Phe Tyr Thr Ser Arg Cys Gly Tyr Arg Leu Cys Ala Arg Ala Tyr Leu
        435                 440                 445

AAC GGG GAC GGG TCG GGG AAG GGA ACG CAC CTG TCC CTG TAC TTT GTG 1392
Asn Gly Asp Gly Ser Gly Lys Gly Thr His Leu Ser Leu Tyr Phe Val
    450                 455                 460

GTG ATG CGC GGT GAG TTT GAC TCG CTG CTG CAG TGG CCG TTC AGG CAG 1440
Val Met Arg Gly Glu Phe Asp Ser Leu Leu Gln Trp Pro Phe Arg Gln
465                 470                 475                 480

AGG GTG ACC CTG ATG CTT TTG GAC CAG AGC GGC AAG AAG AAC CAT ATT 1488
Arg Val Thr Leu Met Leu Leu Asp Gln Ser Gly Lys Lys Asn His Ile
                485                 490                 495

GTG GAG ACC TTC AAA GCT GAC CCC AAC AGC AGC AGC TTC AAA AGG CCA 1536
Val Glu Thr Phe Lys Ala Asp Pro Asn Ser Ser Ser Phe Lys Arg Pro
            500                 505                 510

GAT GGC GAG ATG AAC ATT GCC TCT GGC TGT CCC CGC TTT GTG TCG CAC 1584
Asp Gly Glu Met Asn Ile Ala Ser Gly Cys Pro Arg Phe Val Ser His
        515                 520                 525

TCT ACT CTG GAG AAC TCC AAG AAC ACC TAC ATT AAA GAC GAC ACA CTG 1632
Ser Thr Leu Glu Asn Ser Lys Asn Thr Tyr Ile Lys Asp Asp Thr Leu
    530                 535                 540

TTC TTG AAA GTG GCC GTG GAT TTA ACT GAC TTG GAG GAT CTG 1674
Phe Leu Lys Val Ala Val Asp Leu Thr Asp Leu Glu Asp Leu

545                 550                 555

SEQ ID NO : 3
LENGTH : 2105
TYPE : nucleic acid
STRANDNESS : double
TOPOLOGY : linear
MOLECULE TYPE : cDNA to mRNA
ORIGINAL SOURCE
  ORGANISM : mouse
IMMEDIATE SOURCE
  CLONE: pBSCRAF2(pBSTRAF5)
FEATURE
  Feature Key : CDS
  Location: 188..1861
  Method for the determination of feature : P
SEQUENCE DESCRIPTION

TGTGAGCCGG AGGCGTGTGT GGTAGCGGGC GAACTGAGGC GACGCGGGAC ACCCGCGCCC      60

GGCCGAGGGC ACTTTTGCAA GACTTGTGAG CACAGCCCGT TAACGTGAGC TTAATGCCAG     120

GGTCTCGAGC CTGCGCCGGT GCTATAGCGC GTGCTCGATT GGAAACAGAA CCCGACTCTG     180

CAGAAGA ATG GCT CAT TCG GAG GAG CAA GCG GCT GTC CCC TGC GCC TTC     229
        Met Ala His Ser Glu Glu Gln Ala Ala Val Pro Cys Ala Phe
          1               5                  10

ATC CGC CAG AAC TCT GGC AAC TCA ATT TCC TTG GAC TTT GAG CCC GAC     277
Ile Arg Gln Asn Ser Gly Asn Ser Ile Ser Leu Asp Phe Glu Pro Asp
 15                  20                  25                  30

ACC GAG TAC CAG TTT GTG GAG CAG CTG GAA GAA CGC TAC AAA TGT GCC     325
Thr Glu Tyr Gln Phe Val Glu Gln Leu Glu Glu Arg Tyr Lys Cys Ala
                 35                  40                  45

TTC TGC CAC TCC GTG CTT CAC AAC CCC CAC CAG ACC GGC TGC GGG CAC     373
Phe Cys His Ser Val Leu His Asn Pro His Gln Thr Gly Cys Gly His
             50                  55                  60

CGC TTC TGC CAG CAG TGC ATC CGG TCT CTG AGA GAA TTG AAC TCG GTG     421
Arg Phe Cys Gln Gln Cys Ile Arg Ser Leu Arg Glu Leu Asn Ser Val
         65                  70                  75

CCG ATC TGC CCG GTA GAC AAG GAG GTC ATC AAG CCT CAG GAG GTG TTC     469
Pro Ile Cys Pro Val Asp Lys Glu Val Ile Lys Pro Gln Glu Val Phe
     80                  85                  90

AAA GAC AAC TGC TGC AAA AGA GAA GTT CTC AAT TTA CAC GTC TAC TGC     517
Lys Asp Asn Cys Cys Lys Arg Glu Val Leu Asn Leu His Val Tyr Cys
 95                 100                 105                 110

AAA AAC GCC CCC GGG TGC AAT GCC AGG ATT ATT CTG GGA CGA TTC CAG     565
Lys Asn Ala Pro Gly Cys Asn Ala Arg Ile Ile Leu Gly Arg Phe Gln
                115                 120                 125

GAC CAC CTT CAG CAC TGT TCC TTC CAA GCC GTG CCC TGC CCT AAC GAG     613
Asp His Leu Gln His Cys Ser Phe Gln Ala Val Pro Cys Pro Asn Glu
            130                 135                 140

AGC TGC CGG GAA GCC ATG CTC CGG AAA GAC GTG AAA GAG CAC CTG AGC     661
Ser Cys Arg Glu Ala Met Leu Arg Lys Asp Val Lys Glu His Leu Ser
        145                 150                 155

GCA TAC TGC CGG TTC CGA GAG GAG AAG TGC CTT TAC TGC AAA AGG GAC     709
Ala Tyr Cys Arg Phe Arg Glu Glu Lys Cys Leu Tyr Cys Lys Arg Asp

160                 165                 170

ATA GTG GTG ACC AAC CTG CAG GAT CAT GAG GAA AAC TCG TGT CCT GCG          757
Ile Val Val Thr Asn Leu Gln Asp His Glu Glu Asn Ser Cys Pro Ala
175                 180                 185                 190

TAC CCA GTG TCT TGT CCC AAC AGG TGT GTG CAG ACT ATT CCA AGA GCT          805
Tyr Pro Val Ser Cys Pro Asn Arg Cys Val Gln Thr Ile Pro Arg Ala
                195                 200                 205

AGG GTG AAT GAA CAC CTT ACT GTA TGT CCT GAG GCT GAG CAA GAC TGT          853
Arg Val Asn Glu His Leu Thr Val Cys Pro Glu Ala Glu Gln Asp Cys
            210                 215                 220

CCC TTT AAG CAC TAT GGC TGC ACT GTC AAG GGT AAG CGG GGG AAC TTG          901
Pro Phe Lys His Tyr Gly Cys Thr Val Lys Gly Lys Arg Gly Asn Leu
        225                 230                 235

CTG GAG CAT GAG CGG GCA GCC CTG CAG GAC CAC ATG CTT CTG GTT TTA          949
Leu Glu His Glu Arg Ala Ala Leu Gln Asp His Met Leu Leu Val Leu
    240                 245                 250

GAG AAG AAC TAC CAA CTA GAA CAG CGG ATC TCT GAT TTA TAT CAG AGT          997
Glu Lys Asn Tyr Gln Leu Glu Gln Arg Ile Ser Asp Leu Tyr Gln Ser
255                 260                 265                 270

CTC GAA CAG AAG GAA AGC AAG ATC CAG CAG CTG GCA GAA ACC GTG AAG          1045
Leu Glu Gln Lys Glu Ser Lys Ile Gln Gln Leu Ala Glu Thr Val Lys
                275                 280                 285

AAG TTC GAA AAG GAG CTT AAG CAG TTC ACA CAG ATG TTT GGC AGA AAT          1093
Lys Phe Glu Lys Glu Leu Lys Gln Phe Thr Gln Met Phe Gly Arg Asn
            290                 295                 300

GGA ACT TTC CTC TCA AAT GTC CAG GCT CTC ACC AGT CAC ACG GAC AAG          1141
Gly Thr Phe Leu Ser Asn Val Gln Ala Leu Thr Ser His Thr Asp Lys
        305                 310                 315

TCA GCT TGG CTG GAA GCG CAG GTG CGG CAG CTG CTA CAA ATA GTT AAC          1189
Ser Ala Trp Leu Glu Ala Gln Val Arg Gln Leu Leu Gln Ile Val Asn
    320                 325                 330

CAG CAG CCA AGT CGA CTT GAT CTG AGG TCT TTG GTG GAT GCG GTT GAC          1237
Gln Gln Pro Ser Arg Leu Asp Leu Arg Ser Leu Val Asp Ala Val Asp
335                 340                 345                 350

AGC GTG AAA CAG AGG ATC ACC CAG CTG GAA GCC AGT GAC CAG AGA TTA          1285
Ser Val Lys Gln Arg Ile Thr Gln Leu Glu Ala Ser Asp Gln Arg Leu
                355                 360                 365

GTT CTT TTA GAG GGG GAG ACC AGC AAG CAC GAC GCA CAC ATT AAT ATC          1333
Val Leu Leu Glu Gly Glu Thr Ser Lys His Asp Ala His Ile Asn Ile
            370                 375                 380

CAC AAA GCA CAG CTG AAT AAG AAC GAA GAG CGG TTT AAG CAG CTG GAG          1381
His Lys Ala Gln Leu Asn Lys Asn Glu Glu Arg Phe Lys Gln Leu Glu
        385                 390                 395

GGC GCC TGC TAC AGT GGC AAG CTC ATC TGG AAG GTG ACA GAT TAC AGG          1429
Gly Ala Cys Tyr Ser Gly Lys Leu Ile Trp Lys Val Thr Asp Tyr Arg
    400                 405                 410

GTG AAG AAG AGG GAG GCC GTG GAG GGG CAC ACA GTG TCC GTC TTC AGC          1477
Val Lys Lys Arg Glu Ala Val Glu Gly His Thr Val Ser Val Phe Ser
415                 420                 425                 430

CAG CCT TTC TAC ACC AGC CGC TGC GGC TAC CGG CTC TGT GCC AGG GCG          1525

Gln Pro Phe Tyr Thr Ser Arg Cys Gly Tyr Arg Leu Cys Ala Arg Ala
                435                 440                 445

TAC CTG AAC GGG GAC GGG TCG GGG AAG GGA ACG CAC CTG TCC CTG TAC      1573
Tyr Leu Asn Gly Asp Gly Ser Gly Lys Gly Thr His Leu Ser Leu Tyr
            450                 455                 460

TTT GTG GTG ATG CGC GGT GAG TTT GAC TCG CTG CTG CAG TGG CCG TTC      1621
Phe Val Val Met Arg Gly Glu Phe Asp Ser Leu Leu Gln Trp Pro Phe
        465                 470                 475

AGG CAG AGG GTG ACC CTG ATG CTT TTG GAC CAG AGC GGC AAG AAG AAC      1669
Arg Gln Arg Val Thr Leu Met Leu Leu Asp Gln Ser Gly Lys Lys Asn
    480                 485                 490

CAT ATT GTG GAG ACC TTC AAA GCT GAC CCC AAC AGC AGC AGC TTC AAA      1717
His Ile Val Glu Thr Phe Lys Ala Asp Pro Asn Ser Ser Ser Phe Lys
495                 500                 505                 510

AGG CCA GAT GGC GAG ATG AAC ATT GCC TCT GGC TGT CCC CGC TTT GTG      1765
Arg Pro Asp Gly Glu Met Asn Ile Ala Ser Gly Cys Pro Arg Phe Val
                515                 520                 525

TCG CAC TCT ACT CTG GAG AAC TCC AAG AAC ACC TAC ATT AAA GAC GAC      1813
Ser His Ser Thr Leu Glu Asn Ser Lys Asn Thr Tyr Ile Lys Asp Asp
            530                 535                 540

ACA CTG TTC TTG AAA GTG GCC GTG GAT TTA ACT GAC TTG GAG GAT CTG      1861
Thr Leu Phe Leu Lys Val Ala Val Asp Leu Thr Asp Leu Glu Asp Leu
        545                 550                 555

TAGTGTTACC TGATAAGGAA ACTTCTCAGC ACAGGAAAAG GTGTGGCTGT CCCTTGGGCG      1921

CAGCCCTCTG GACTGAGCAG GCTCTTGTTC TTGTCTTCCT GCCTCCGATG TCTGATGTGT      1981

CATCTTTTTA TCTTGGATCC TTCCCTGGTT TGAAACTTTA AACTCTTGAA ATATTGCTGT      2041

TATTTATATT TTTGTATCTT CCAAAAAATT ATAATAATTT GACAACAAAA AAAAAAAAAA      2101

AAAA                                                                   2105

SEQ ID NO : 4
LENGTH : 557
TYPE : amino acid
TOPOLOGY : linear
MOLECULE TYPE : peptide
ORIGINAL SOURCE
  ORGANISM : human
SEQUENCE DESCRIPTION

Met Ala Tyr Ser Glu Glu His Lys Gly Met Pro Cys Gly Phe Ile Arg
  1               5                  10                  15

Gln Asn Ser Gly Asn Ser Ile Ser Leu Asp Phe Glu Pro Ser Ile Glu
            20                  25                  30

Tyr Gln Phe Val Glu Arg Leu Glu Glu Arg Tyr Lys Cys Ala Phe Cys
        35                  40                  45

His Ser Val Leu His Asn Pro His Gln Thr Gly Cys Gly His Arg Phe
    50                  55                  60

Cys Gln His Cys Ile Leu Ser Leu Arg Glu Leu Asn Thr Val Pro Ile
 65                  70                  75                  80

Cys Pro Val Asp Lys Glu Val Ile Lys Ser Gln Glu Val Phe Lys Asp
                85                  90                  95

Asn Cys Cys Lys Arg Glu Val Leu Asn Leu Tyr Val Tyr Cys Ser Asn
            100                 105                 110

Ala Pro Gly Cys Asn Ala Lys Val Ile Leu Gly Arg Tyr Gln Asp His
        115                 120                 125

Leu Gln Gln Cys Leu Phe Gln Pro Val Gln Cys Ser Asn Glu Lys Cys
    130                 135                 140

Arg Glu Pro Val Leu Arg Lys Asp Leu Lys Glu His Leu Ser Ala Ser
145                 150                 155                 160

Cys Gln Phe Arg Lys Glu Lys Cys Leu Tyr Cys Lys Lys Asp Val Val
                165                 170                 175

Val Ile Asn Leu Gln Asn His Glu Glu Asn Leu Cys Pro Glu Tyr Pro
            180                 185                 190

Val Phe Cys Pro Asn Asn Cys Ala Lys Ile Ile Leu Lys Thr Glu Val
        195                 200                 205

Asp Glu His Leu Ala Val Cys Pro Glu Ala Glu Gln Asp Cys Pro Phe
    210                 215                 220

Lys His Tyr Gly Cys Ala Val Thr Asp Lys Arg Arg Asn Leu Gln Gln
225                 230                 235                 240

His Glu His Ser Ala Leu Arg Glu His Met Arg Leu Val Leu Glu Lys
                245                 250                 255

Asn Val Gln Leu Glu Glu Gln Ile Ser Asp Leu His Lys Ser Leu Glu
            260                 265                 270

Gln Lys Glu Ser Lys Ile Gln Gln Leu Ala Glu Thr Ile Lys Lys Leu
        275                 280                 285

Glu Lys Glu Phe Lys Gln Phe Ala Gln Leu Phe Gly Lys Asn Gly Ser
    290                 295                 300

Phe Leu Pro Asn Ile Gln Val Phe Ala Ser His Ile Asp Lys Ser Ala
305                 310                 315                 320

Trp Leu Glu Ala Gln Val His Gln Leu Leu Gln Met Val Asn Gln Gln
                325                 330                 335

Gln Asn Lys Phe Asp Leu Arg Pro Leu Met Glu Ala Val Asp Thr Val
            340                 345                 350

Lys Gln Lys Ile Thr Leu Leu Glu Asn Asn Asp Gln Arg Leu Ala Val
        355                 360                 365

Leu Glu Glu Glu Thr Asn Lys His Asp Thr His Ile Asn Ile His Lys
    370                 375                 380

Ala Gln Leu Ser Lys Asn Glu Glu Arg Phe Lys Leu Leu Glu Gly Thr
385                 390                 395                 400

Cys Tyr Asn Gly Lys Leu Ile Trp Lys Val Thr Asp Tyr Lys Met Lys
                405                 410                 415

Lys Arg Glu Ala Val Asp Gly His Thr Val Ser Ile Phe Ser Gln Ser
            420                 425                 430

Phe Tyr Thr Ser Arg Cys Gly Tyr Arg Leu Cys Ala Arg Ala Tyr Leu

435                 440                 445

Asn Gly Asp Gly Ser Gly Arg Gly Ser His Leu Ser Leu Tyr Phe Val
    450                 455                 460

Val Met Arg Gly Glu Phe Asp Ser Leu Leu Gln Trp Pro Phe Arg Gln
465                 470                 475                 480

Arg Val Thr Leu Met Leu Leu Asp Gln Ser Gly Lys Lys Asn Ile Met
                485                 490                 495

Glu Thr Phe Lys Pro Asp Pro Asn Ser Ser Ser Phe Lys Arg Pro Asp
            500                 505                 510

Gly Glu Met Asn Ile Ala Ser Gly Cys Pro Arg Phe Val Ala His Ser
        515                 520                 525

Val Leu Glu Asn Ala Lys Asn Ala Tyr Ile Lys Asp Asp Thr Leu Phe
    530                 535                 540

Leu Lys Val Ala Val Asp Leu Thr Asp Leu Glu Asp Leu
545                 550                 555

SEQ ID NO : 5
LENGTH : 1671
TYPE : nucleic acid
STRANDNESS : double
TOPOLOGY : linear
MOLECULE TYPE : cDNA to mRNA
ORIGINAL SOURCE
  ORGANISM : human
FEATURE
  Feature Key : CDS
  Location: 1..1671
  Method for the determination of feature : P
SEQUENCE DESCRIPTION

ATG GCT TAT TCA GAA GAG CAT AAA GGT ATG CCC TGT GGT TTC ATC CGC          48
Met Ala Tyr Ser Glu Glu His Lys Gly Met Pro Cys Gly Phe Ile Arg
  1               5                  10                  15

CAG AAT TCC GGC AAC TCC ATT TCC TTG GAC TTT GAG CCC AGT ATA GAG          96
Gln Asn Ser Gly Asn Ser Ile Ser Leu Asp Phe Glu Pro Ser Ile Glu
             20                  25                  30

TAC CAG TTT GTG GAG CGG TTG GAA GAG CGC TAC AAA TGT GCC TTC TGC         144
Tyr Gln Phe Val Glu Arg Leu Glu Glu Arg Tyr Lys Cys Ala Phe Cys
         35                  40                  45

CAC TCG GTG CTT CAC AAC CCC CAC CAG ACA GGA TGT GGG CAC CGC TTC         192
His Ser Val Leu His Asn Pro His Gln Thr Gly Cys Gly His Arg Phe
     50                  55                  60

TGC CAG CAC TGC ATC CTG TCC CTG AGA GAA TTA AAC ACA GTG CCA ATC         240
Cys Gln His Cys Ile Leu Ser Leu Arg Glu Leu Asn Thr Val Pro Ile
 65                  70                  75                  80

TGC CCT GTA GAT AAA GAG GTC ATC AAA TCT CAG GAG GTT TTT AAA GAC         288
Cys Pro Val Asp Lys Glu Val Ile Lys Ser Gln Glu Val Phe Lys Asp
                 85                  90                  95

AAT TGT TGC AAA AGA GAA GTC CTC AAC TTA TAT GTA TAT TGC AGC AAT         336
Asn Cys Cys Lys Arg Glu Val Leu Asn Leu Tyr Val Tyr Cys Ser Asn
            100                 105                 110

GCT CCT GGA TGT AAT GCC AAG GTT ATT CTG GGC CGG TAC CAG GAT CAC         384

Ala Pro Gly Cys Asn Ala Lys Val Ile Leu Gly Arg Tyr Gln Asp His
        115                 120                 125

CTT CAG CAG TGC TTA TTT CAA CCT GTG CAG TGT TCT AAT GAG AAG TGC     432
Leu Gln Gln Cys Leu Phe Gln Pro Val Gln Cys Ser Asn Glu Lys Cys
    130                 135                 140

CGG GAG CCA GTC CTA CGG AAA GAC CTG AAA GAG CAT TTG AGT GCA TCC     480
Arg Glu Pro Val Leu Arg Lys Asp Leu Lys Glu His Leu Ser Ala Ser
145                 150                 155                 160

TGT CAG TTT CGA AAG GAA AAA TGC CTT TAT TGC AAA AAG GAT GTG GTA     528
Cys Gln Phe Arg Lys Glu Lys Cys Leu Tyr Cys Lys Lys Asp Val Val
                165                 170                 175

GTC ATC AAT CTA CAG AAT CAT GAG GAA AAC TTG TGT CCT GAA TAC CCA     576
Val Ile Asn Leu Gln Asn His Glu Glu Asn Leu Cys Pro Glu Tyr Pro
            180                 185                 190

GTA TTT TGT CCC AAC AAT TGT GCG AAG ATT ATT CTA AAA ACT GAG GTA     624
Val Phe Cys Pro Asn Asn Cys Ala Lys Ile Ile Leu Lys Thr Glu Val
        195                 200                 205

GAT GAA CAC CTG GCT GTA TGT CCT GAA GCT GAG CAA GAC TGT CCT TTT     672
Asp Glu His Leu Ala Val Cys Pro Glu Ala Glu Gln Asp Cys Pro Phe
    210                 215                 220

AAG CAC TAT GGC TGT GCT GTA ACG GAT AAA CGG AGG AAC CTG CAG CAA     720
Lys His Tyr Gly Cys Ala Val Thr Asp Lys Arg Arg Asn Leu Gln Gln
225                 230                 235                 240

CAT GAG CAT TCA GCC TTA CGG GAG CAC ATG CGT TTG GTT TTA GAA AAG     768
His Glu His Ser Ala Leu Arg Glu His Met Arg Leu Val Leu Glu Lys
                245                 250                 255

AAT GTC CAA TTA GAA GAA CAG ATT TCT GAC TTA CAC AAG AGC CTA GAA     816
Asn Val Gln Leu Glu Glu Gln Ile Ser Asp Leu His Lys Ser Leu Glu
            260                 265                 270

CAG AAA GAA AGT AAA ATC CAG CAG CTA GCA GAA ACT ATA AAG AAA CTT     864
Gln Lys Glu Ser Lys Ile Gln Gln Leu Ala Glu Thr Ile Lys Lys Leu
        275                 280                 285

GAA AAG GAG TTC AAG CAG TTT GCA CAG TTG TTT GGC AAA AAT GGA AGC     912
Glu Lys Glu Phe Lys Gln Phe Ala Gln Leu Phe Gly Lys Asn Gly Ser
    290                 295                 300

TTC CTC CCA AAC ATC CAG GTT TTT GCC AGT CAC ATT GAC AAG TCA GCT     960
Phe Leu Pro Asn Ile Gln Val Phe Ala Ser His Ile Asp Lys Ser Ala
305                 310                 315                 320

TGG CTA GAA GCT CAA GTG CAT CAA TTA TTA CAA ATG GTT AAC CAG CAA    1008
Trp Leu Glu Ala Gln Val His Gln Leu Leu Gln Met Val Asn Gln Gln
                325                 330                 335

CAA AAT AAA TTT GAC CTG AGA CCT TTG ATG GAA GCA GTT GAT ACA GTG    1056
Gln Asn Lys Phe Asp Leu Arg Pro Leu Met Glu Ala Val Asp Thr Val
            340                 345                 350

AAA CAG AAA ATT ACC CTG CTA GAA AAC AAT GAT CAA AGA TTA GCC GTT    1104
Lys Gln Lys Ile Thr Leu Leu Glu Asn Asn Asp Gln Arg Leu Ala Val
        355                 360                 365

TTA GAA GAG GAA ACT AAC AAA CAT GAT ACC CAC ATT AAT ATT CAT AAA    1152
Leu Glu Glu Glu Thr Asn Lys His Asp Thr His Ile Asn Ile His Lys
    370                 375                 380

GCA CAG CTG AGT AAA AAT GAA GAG CGA TTT AAA CTG CTG GAG GGT ACT 1200
Ala Gln Leu Ser Lys Asn Glu Glu Arg Phe Lys Leu Leu Glu Gly Thr
385                 390                 395                 400

TGC TAT AAT GGA AAG CTC ATT TGG AAG GTG ACA GAT TAC AAG ATG AAG 1248
Cys Tyr Asn Gly Lys Leu Ile Trp Lys Val Thr Asp Tyr Lys Met Lys
                405                 410                 415

AAG AGA GAG GCG GTG GAT GGG CAC ACA GTG TCC ATC TTC AGC CAG TCC 1296
Lys Arg Glu Ala Val Asp Gly His Thr Val Ser Ile Phe Ser Gln Ser
            420                 425                 430

TTC TAC ACC AGC CGC TGT GGC TAC CGG CTC TGT GCT AGA GCA TAC CTG 1344
Phe Tyr Thr Ser Arg Cys Gly Tyr Arg Leu Cys Ala Arg Ala Tyr Leu
        435                 440                 445

AAT GGG GAT GGG TCA GGG AGG GGG TCA CAC CTG TCC CTA TAC TTT GTG 1392
Asn Gly Asp Gly Ser Gly Arg Gly Ser His Leu Ser Leu Tyr Phe Val
    450                 455                 460

GTC ATG CGA GGA GAG TTT GAC TCA CTG TTG CAG TGG CCA TTC AGG CAG 1440
Val Met Arg Gly Glu Phe Asp Ser Leu Leu Gln Trp Pro Phe Arg Gln
465                 470                 475                 480

AGG GTG ACC CTG ATG CTT CTG GAC CAG AGT GGC AAA AAG AAC ATT ATG 1488
Arg Val Thr Leu Met Leu Leu Asp Gln Ser Gly Lys Lys Asn Ile Met
                485                 490                 495

GAG ACC TTC AAA CCT GAC CCC AAT AGC AGC AGC TTT AAA AGA CCT GAT 1536
Glu Thr Phe Lys Pro Asp Pro Asn Ser Ser Ser Phe Lys Arg Pro Asp
            500                 505                 510

GGG GAG ATG AAC ATT GCA TCT GGC TGT CCC CGC TTT GTG GCT CAT TCT 1584
Gly Glu Met Asn Ile Ala Ser Gly Cys Pro Arg Phe Val Ala His Ser
        515                 520                 525

GTT TTG GAG AAT GCC AAG AAC GCC TAC ATT AAA GAT GAC ACT CTG TTC 1632
Val Leu Glu Asn Ala Lys Asn Ala Tyr Ile Lys Asp Asp Thr Leu Phe
    530                 535                 540

TTG AAA GTG GCC GTG GAC TTA ACT GAC CTG GAG GAT CTC 1671
Leu Lys Val Ala Val Asp Leu Thr Asp Leu Glu Asp Leu
545                 550                 555

SEQ ID NO : 6
LENGTH : 3993
TYPE : nucleic acid
STRANDNESS : double
TOPOLOGY : linear
MOLECULE TYPE : cDNA to mRNA
ORIGINAL SOURCE
ORGANISM : human
IMMEDIATE SOURCE
CLONE: pBShTRAF5
FEATURE
Feature Key : CDS
Location: 55..1725
Method for the determination of feature : P
SEQUENCE DESCRIPTION

GCAGCAGCCG CGCCTGCAGA CCGGCCTCGC GGAGCCCGCG CGCCGAGCCC CACA ATG 57
Met
1

GCT TAT TCA GAA GAG CAT AAA GGT ATG CCC TGT GGT TTC ATC CGC CAG 105

Ala Tyr Ser Glu Glu His Lys Gly Met Pro Cys Gly Phe Ile Arg Gln
                5                   10                  15

AAT TCC GGC AAC TCC ATT TCC TTG GAC TTT GAG CCC AGT ATA GAG TAC     153
Asn Ser Gly Asn Ser Ile Ser Leu Asp Phe Glu Pro Ser Ile Glu Tyr
        20                  25                  30

CAG TTT GTG GAG CGG TTG GAA GAG CGC TAC AAA TGT GCC TTC TGC CAC     201
Gln Phe Val Glu Arg Leu Glu Glu Arg Tyr Lys Cys Ala Phe Cys His
    35                  40                  45

TCG GTG CTT CAC AAC CCC CAC CAG ACA GGA TGT GGG CAC CGC TTC TGC     249
Ser Val Leu His Asn Pro His Gln Thr Gly Cys Gly His Arg Phe Cys
50                  55                  60                  65

CAG CAC TGC ATC CTG TCC CTG AGA GAA TTA AAC ACA GTG CCA ATC TGC     297
Gln His Cys Ile Leu Ser Leu Arg Glu Leu Asn Thr Val Pro Ile Cys
                70                  75                  80

CCT GTA GAT AAA GAG GTC ATC AAA TCT CAG GAG GTT TTT AAA GAC AAT     345
Pro Val Asp Lys Glu Val Ile Lys Ser Gln Glu Val Phe Lys Asp Asn
            85                  90                  95

TGT TGC AAA AGA GAA GTC CTC AAC TTA TAT GTA TAT TGC AGC AAT GCT     393
Cys Cys Lys Arg Glu Val Leu Asn Leu Tyr Val Tyr Cys Ser Asn Ala
        100                 105                 110

CCT GGA TGT AAT GCC AAG GTT ATT CTG GGC CGG TAC CAG GAT CAC CTT     441
Pro Gly Cys Asn Ala Lys Val Ile Leu Gly Arg Tyr Gln Asp His Leu
    115                 120                 125

CAG CAG TGC TTA TTT CAA CCT GTG CAG TGT TCT AAT GAG AAG TGC CGG     489
Gln Gln Cys Leu Phe Gln Pro Val Gln Cys Ser Asn Glu Lys Cys Arg
130                 135                 140                 145

GAG CCA GTC CTA CGG AAA GAC CTG AAA GAG CAT TTG AGT GCA TCC TGT     537
Glu Pro Val Leu Arg Lys Asp Leu Lys Glu His Leu Ser Ala Ser Cys
                150                 155                 160

CAG TTT CGA AAG GAA AAA TGC CTT TAT TGC AAA AAG GAT GTG GTA GTC     585
Gln Phe Arg Lys Glu Lys Cys Leu Tyr Cys Lys Lys Asp Val Val Val
            165                 170                 175

ATC AAT CTA CAG AAT CAT GAG GAA AAC TTG TGT CCT GAA TAC CCA GTA     633
Ile Asn Leu Gln Asn His Glu Glu Asn Leu Cys Pro Glu Tyr Pro Val
        180                 185                 190

TTT TGT CCC AAC AAT TGT GCG AAG ATT ATT CTA AAA ACT GAG GTA GAT     681
Phe Cys Pro Asn Asn Cys Ala Lys Ile Ile Leu Lys Thr Glu Val Asp
    195                 200                 205

GAA CAC CTG GCT GTA TGT CCT GAA GCT GAG CAA GAC TGT CCT TTT AAG     729
Glu His Leu Ala Val Cys Pro Glu Ala Glu Gln Asp Cys Pro Phe Lys
210                 215                 220                 225

CAC TAT GGC TGT GCT GTA ACG GAT AAA CGG AGG AAC CTG CAG CAA CAT     777
His Tyr Gly Cys Ala Val Thr Asp Lys Arg Arg Asn Leu Gln Gln His
                230                 235                 240

GAG CAT TCA GCC TTA CGG GAG CAC ATG CGT TTG GTT TTA GAA AAG AAT     825
Glu His Ser Ala Leu Arg Glu His Met Arg Leu Val Leu Glu Lys Asn
            245                 250                 255

GTC CAA TTA GAA GAA CAG ATT TCT GAC TTA CAC AAG AGC CTA GAA CAG     873
Val Gln Leu Glu Glu Gln Ile Ser Asp Leu His Lys Ser Leu Glu Gln
        260                 265                 270

AAA GAA AGT AAA ATC CAG CAG CTA GCA GAA ACT ATA AAG AAA CTT GAA 921
Lys Glu Ser Lys Ile Gln Gln Leu Ala Glu Thr Ile Lys Lys Leu Glu
    275                 280                 285

AAG GAG TTC AAG CAG TTT GCA CAG TTG TTT GGC AAA AAT GGA AGC TTC 969
Lys Glu Phe Lys Gln Phe Ala Gln Leu Phe Gly Lys Asn Gly Ser Phe
290                 295                 300                 305

CTC CCA AAC ATC CAG GTT TTT GCC AGT CAC ATT GAC AAG TCA GCT TGG 1017
Leu Pro Asn Ile Gln Val Phe Ala Ser His Ile Asp Lys Ser Ala Trp
                310                 315                 320

CTA GAA GCT CAA GTG CAT CAA TTA TTA CAA ATG GTT AAC CAG CAA CAA 1065
Leu Glu Ala Gln Val His Gln Leu Leu Gln Met Val Asn Gln Gln Gln
            325                 330                 335

AAT AAA TTT GAC CTG AGA CCT TTG ATG GAA GCA GTT GAT ACA GTG AAA 1113
Asn Lys Phe Asp Leu Arg Pro Leu Met Glu Ala Val Asp Thr Val Lys
        340                 345                 350

CAG AAA ATT ACC CTG CTA GAA AAC AAT GAT CAA AGA TTA GCC GTT TTA 1161
Gln Lys Ile Thr Leu Leu Glu Asn Asn Asp Gln Arg Leu Ala Val Leu
    355                 360                 365

GAA GAG GAA ACT AAC AAA CAT GAT ACC CAC ATT AAT ATT CAT AAA GCA 1209
Glu Glu Glu Thr Asn Lys His Asp Thr His Ile Asn Ile His Lys Ala
370                 375                 380                 385

CAG CTG AGT AAA AAT GAA GAG CGA TTT AAA CTG CTG GAG GGT ACT TGC 1257
Gln Leu Ser Lys Asn Glu Glu Arg Phe Lys Leu Leu Glu Gly Thr Cys
                390                 395                 400

TAT AAT GGA AAG CTC ATT TGG AAG GTG ACA GAT TAC AAG ATG AAG AAG 1305
Tyr Asn Gly Lys Leu Ile Trp Lys Val Thr Asp Tyr Lys Met Lys Lys
            405                 410                 415

AGA GAG GCG GTG GAT GGG CAC ACA GTG TCC ATC TTC AGC CAG TCC TTC 1353
Arg Glu Ala Val Asp Gly His Thr Val Ser Ile Phe Ser Gln Ser Phe
        420                 425                 430

TAC ACC AGC CGC TGT GGC TAC CGG CTC TGT GCT AGA GCA TAC CTG AAT 1401
Tyr Thr Ser Arg Cys Gly Tyr Arg Leu Cys Ala Arg Ala Tyr Leu Asn
    435                 440                 445

GGG GAT GGG TCA GGG AGG GGG TCA CAC CTG TCC CTA TAC TTT GTG GTC 1449
Gly Asp Gly Ser Gly Arg Gly Ser His Leu Ser Leu Tyr Phe Val Val
450                 455                 460                 465

ATG CGA GGA GAG TTT GAC TCA CTG TTG CAG TGG CCA TTC AGG CAG AGG 1497
Met Arg Gly Glu Phe Asp Ser Leu Leu Gln Trp Pro Phe Arg Gln Arg
                470                 475                 480

GTG ACC CTG ATG CTT CTG GAC CAG AGT GGC AAA AAG AAC ATT ATG GAG 1545
Val Thr Leu Met Leu Leu Asp Gln Ser Gly Lys Lys Asn Ile Met Glu
            485                 490                 495

ACC TTC AAA CCT GAC CCC AAT AGC AGC AGC TTT AAA AGA CCT GAT GGG 1593
Thr Phe Lys Pro Asp Pro Asn Ser Ser Ser Phe Lys Arg Pro Asp Gly
        500                 505                 510

GAG ATG AAC ATT GCA TCT GGC TGT CCC CGC TTT GTG GCT CAT TCT GTT 1641
Glu Met Asn Ile Ala Ser Gly Cys Pro Arg Phe Val Ala His Ser Val
    515                 520                 525

TTG GAG AAT GCC AAG AAC GCC TAC ATT AAA GAT GAC ACT CTG TTC TTG 1689
Leu Glu Asn Ala Lys Asn Ala Tyr Ile Lys Asp Asp Thr Leu Phe Leu
530                 535                 540                 545

AAA GTG GCC GTG GAC TTA ACT GAC CTG GAG GAT CTC TAGTCACTGT 1735
Lys Val Ala Val Asp Leu Thr Asp Leu Glu Asp Leu
                550                 555

TATGGGGTGA TAAGAGGACT TCTTGGGGCC AGAACTGTGG AGGAGAGCAC ATTTGATTAT 1795

CATATTGACC TGGATTTAGA CTCAAAGCAC ATTTGTATTT GCCTTTTTCC TTAACGTTTG 1855

AAGTCAGTTT AAAACTTCTG AAGTGCTGTC TTTTTACATT TTACTCTGTC CCAGTTTGAA 1915

ACTTAAAACT CTTAGAATAT TCTCTTATTA TTTATATTTT TATATTTCTT GAAAGATGGT 1975

AAGTTTCTTG AAGTTTTTGG GGCGTTTCTC TTTTACTGGT GCTTAGCGCA GTGTCTCGGG 2035

CACTCTAAAT ATTGAGTGTT ATGGAGGACA CAGAGGTAGC AGAATCCCAG TTGAAAATGT 2095

TTTGATATTT TATTGTTTGG CCTATTGATT CTAGACCTGG CCTTAAGTCT GCAAAAGCCA 2155

TCTTTATAAG GTAGGCTGTT CCAGTTAAGA AGTGGGTGAT GTAGTTACAA AGATAATATG 2215

CTCAGTTTGG ACCTTTTTTT CAGTTAAATG CTAAATATAT GAAAATTACT ATACCTCTAA 2275

GTATTTTCAT GAAATTCACC AGCAGTTTGC AAGCACAGTT TTGCAAGGCT GCATAAGAAC 2335

TGGTGAATGG GGTAAGCATT TTCATTCTTC CTGCTGAAGT AAAGCAGAAA GTACTGCATA 2395

GTATATGAGA TATAGCCAGC TAGCTAAAGT TCAGATTTTG TTAGGTTCAA CCCTATGAAA 2455

AAAACTATTT TCATAGGTCA AAAATGGTAA AAAATTAGCA GTTTCATAAG ATTCAACCAA 2515

ATAAATATAT ATATACACAC ACACATACAT ATACACCTAT ATATGTGTGT ATACAAACAG 2575

TTCGAATGTA TTTTGGTGAC AGTAATAAAT CAATGTGAGG ATGGATAGAA TTTAGTATAT 2635

GATAGAGAAA ATGTCATAAA TGGATAAAAG GAATTTACAA CTTGAGGAGA AAACCTTTAC 2695

AATTTCCTAT GGGTGTCAGA AGTACTCTCA GCGAAAACTG ATGGCTAAAA CAGTATCTAC 2755

TATTCTCTGA TAACTTTTTT TTTGAGACAG AGTTTCATTG TCACCCAGGC TGGAGTACAG 2815

TGGCATGATC TCAGCTCACT GCAAACTCTG CCTCCCGAAT TCAAGTGATT CTCCTGCCTC 2875

AGCCTCCTGA GTAGCTGGGA TTACAGGCGC CCGTCACCAC ACCCAGGTAA TTTTTGTATT 2935

TTTAGTAGAG ACGGAGTTTT GCCATGTTGG CCAAGCTGAT CTCAAACTCC TGACCTCAAG 2995

TGATCTGCCC GCCTCGGCCT CCCAAAGTGC TGAGATTACA GGCATGACCC ACCGCGTCAA 3055

GCCTCTGACA ACTATTGAAT TTGTAAGCTG CTATGCAAAT GGGCATTTAT ATAAACTTGT 3115

GATGTTTCTT GTCAGAATTC TGAGTACTCT GTGAAGAACA GAAATGATCA TATTCTTATG 3175

CATCTATCTG TATGGGTCTG AAGGTGTATA TACAAACTGA GATGAGTCCT TATGACTCTT 3235

GATAAGCCTG AGTTTAACAA CAACAAAAAT GCCAAGTTGT CCTGAGCCCT TCTGCGTTGT 3295

TATGCCACTT CCCTACTGCT CATATGCACG CTGGCTCCCC TGGGCACGCA AGGATGAGTA 3355

TGGGCCATGG GCCCCTGTAG AGCTGCTTAC CTGGTGATGA CCATGCACCT TACAATTTCT 3415

GAACAGTTAA CCCTATAGAA GCATGCTTTA TATGAGTGTC TTCTGGGAAG AGGAACCTTC 3475

TTAATCTCTT CTGTGGGATT TTCAAAATGC TAAAGACTCA CACTGCAGCA ATCATCCCAG 3535

ATGATTAAAT TCAAAGAAAT AGGTTCACAA CAGGAATATA CTGAAGAACT AGAGTGTCAC 3595

TGCTGGTGAA CTGTGGCACG GTTGCTCAAC ACATCACCTC GGACAAATTC AGGAAGCATT 3655
TCTTTAGCCC ACAAGTCCAG ACCCAGGTGC TCTGTATGTT TGTTTTTAAT ATTCATCATA 3715
TCCAAGTTCA CTCTGTCTTC CTGAGCAGTG GAAGATCATA TTGCTGTAAC TTCTTTTAAG 3775
TAGTTGATGT GGAAAACATT TTAAAGTGAA TTTGTCAAAA TGCTGGTTTT GTGTTTTATC 3835
CAACTTTTGT GCATATATAT AAAGTATGTC ATGGCATGGT TTGCTTAGGA GTTCAGAGTT 3895
CCTTCATCAT CGAAATAGTG ATTAAGTGAT CCCAGAACAA GGAATACTAG AGTAAAAAGC 3955
ACCTCTTTTT CAGAAAAAAA AAAAAAAAAA AAAAAAAA 3993

## Claims

1. TRAF5 protein associating with the intracellular domain of CD40.

2. A polypeptide comprising at least one of the polypeptides having an amino acid sequence shown as No.45-84, No.110-249, No.251-403 or No.404-558 of the SEQ ID No.1 in the Sequence Listing.

3. A polypeptide comprising at least one of the polypeptides having an amino acid sequence shown as No.45-84, No.110-249, No.251-403 or No.404-557 of the SEQ ID No.4 of the Sequence Listing.

4. A polypeptide comprising the polypeptide of the SEQ ID No.1 in the Sequence Listing.

5. A polypeptide comprising the polypeptide of the SEQ ID No.4 in the Sequence Listing.

6. A polypeptide consisting of the polypeptide of the SEQ ID No.1 in the Sequence Listing or any part thereof.

7. A polypeptide consisting of the polypeptide of the SEQ ID No.4 in the Sequence Listing or any part thereof.

8. A DNA comprising the base sequence encoding the polypeptide comprising at least one of the polypeptides having an amino acid sequence shown as No.45-84, No.110-249, No.251-403 or No.404-558 of the SEQ ID No.1 in the Sequence Listing.

9. A DNA comprising the base sequence encoding the polypeptide comprising at least one of the polypeptides having an amino acid sequence shown as No.45-84, No.110-249, No.251-403 or No.404-557 of the SEQ ID No.4 of the Sequence Listing.

10. A DNA comprising the base sequence encoding the polypeptide of Claim 6.

11. A DNA comprising the base sequence encoding the polypeptlde of Claim 7.

12. A DNA comprising the base sequence of the SEQ ID No.2 in the sequence Listing or any part thereof.

13. A DNA comprising the base sequence of the SEQ ID No.5 in the Sequence Listing or any part thereof.

14. An antisense oligonucleotide and its derivatives for the DNA of Claim 8, 10 or 12.

15. An antisense oligonucleotide and its derivatives for the DNA of Claim 9, 11 or 13.

16. An antibody which recognizes the TRAF5 of Claim 1.

17. An antibody which recognizes the polypeptide of Claim 2, 4 or 6.

18. An antibody which recognizes the polypeptide of Claim 3, 5 or 7.

19. An antibody of Claim 16, 17 or 18, which inhibits CD40-mediated signal transduction.

20. A monoclonal antibody of Claim 16, 17, 18 or 19.

21. A vector comprising the DNA of Claim 8, 10 or 12.

22. A vector comprising the DNA of Claim 9, 11 or 13.

23. A transformant which is transformed by the vector of Claim 21.

24. A transformant which is transformed by the vector of Claim 22.

25. A method for the production of TRAF5 or the polypeptide, comprising culturing the transformant of Claim 23.

26. A method for the production of TRAF5 or the polypeptide, comprising culturing the transformant of Claim 24.

27. A method for the screening of the substance which binds to TRAF5 protein of Claim 1, or the polypeptide of one of Claim 2 to 7, or regulates the activity or the expression of TRAF5 protein of Claim 1, or the polypeptide of one of Claim 2 to 7, using said TRAF5 protein, said polypeptide, or the antibody of one of Claim 16 to 18.

28. The substances obtained by the screening method of Claim 27, which binds to TRAF5 protein of Claim 1 or the polypeptide of one of Claim 2 to 7, or regulates their activity or expression.

29. A medical composition used for the treatment of immune diseases, comprising the TRAF5 protein of Claim 1 or the polypeptide of one of Claim 2 to 7 as an effective ingredient.

30. A medical composition used for the treatment of allergy, comprising the TRAF5 protein of Claim 1 or the polypeptide of one of Claim 2 to 7 as an effective ingredient.

31. A medical composition with cell growth-inhibiting activity, comprising the TRAF5 protein of Claim 1 or the polypeptide of one of Claim 2 to 7 as an effective ingredient.

32. A medical composition used for the treatment of immune diseases, comprising the antisense oligonucleotide of Claim 14 or 15 or its derivatives as an effective ingredient.

33. A medical composition used for the treatment of allergy, comprising the antisense oligonucleotide of Claim 14 or 15 or its derivatives as an effective ingredient.

34. A medical composition with cell growth-inhibiting activity, comprising the antisense oligonucleotide of Claim 14 or 15 or its derivatives as an effective ingredient.

35. A medical composition used for the treatment of immune diseases, comprising the antibody of one of Claim 16 to 20 as an effective ingredient.

36. A medical composition used for the treatment of allergy, comprising the antibody of one of Claim 16 to 20 as an effective ingredient.

37. A medical composition with cell growth-inhibiting activity, comprising the antibody of one of Claim 16 to 20 as an effective ingredient.

38. A medical composition used for the treatment of immune diseases, comprising the substance of Claim 28 as an effective ingredient.

39. A medical composition used for the treatment of allergy, comprising the substance of Claim 28 as an effective ingredient.

40. A medical composition with cell growth-inhibiting activity, comprising the substance of Claim 28 as an effective ingredient.
